# EUROPEAN PATENT SPECIFICATION

(11) **EP 1 085 908 B1**
(45) Date of publication and mention of the grant of the patent: **02.09.2009**
(21) Application number: 99930180.7
(22) Date of filing: 08.06.1999
(51) Int. Cl.: A61K 47/48

(54) **RECOMBINANT IMMUNOTOXIN DIRECTED AGAINST THE HIV-1 GP120 ENVELOPE GLYCOPROTEIN**
GEGEN HIV-1 GP120 ENVELOPE GLYKOPROTEIN GERICHTETE REKOMBINANT-IMMUNOTOXIN
IMMUNOTOXINE DE RECOMBINAISON DIRIGEE VERS LA GLYCOPROTEINE DE L'ENVELOPPE GP120 DU VIH

(30) Priority: 11.06.1998 US 88860 P
(43) Date of publication of application: 28.03.2001
(73) Proprietor: THE GOVERNMENT OF THE UNITED STATES OF AMERICA as represented by the SECRETARY OF THE DEPARTMENT OF HEALTH AND HUMAN SERVICES, Bethesda, MD 20892-9902 (US); THE SCRIPPS RESEARCH INSTITUTE, La Jolla, CA 92037 (US)
(72) Inventor: PASTAN, Ira, H., Potomac, MD 20854 (US); BERA, Tapan, K., Gaithersberg, MD 20877 (US); KENNEDY, Paul, E., Silver Spring, MD 20910 (US); BERGER, Edward, A., Rockville, MD 20852 (US); BARBAS, Carlos, F., III, San Diego, CA 92122 (US)
(74) Representative: Campbell, Patrick John Henry
(86) International application number: PCT/US1999/012909
(87) International publication number: WO 1999/064073

(56) References cited:
- WO-A-97/13529
- US-A- 5 458 878
- US-A- 5 834 599
- DATABASE CHEMABS [Online] CHEMICAL ABSTRACTS SERVICE, COLUMBUS, OHIO, US BERA, TAPAN K. ET AL: "Specific killing of HIV-infected lymphocytes by a recombinant immunotoxin directed against the HIV-1 envelope glycoprotein" retrieved from STN Database accession no. 129:229342 XP002125548 & MOL. MED. (N. Y.) (1998), 4(6), 384-391 ,
- DATABASE CHEMABS [Online] CHEMICAL ABSTRACTS SERVICE, COLUMBUS, OHIO, US BERA, TAPAN K. ET AL: "A bivalent disulfide-stabilized Fv with improved antigen binding to erbB2" retrieved from STN Database accession no. 129:270188 XP002125549 & J. MOL. BIOL. (1998), 281(3), 475-483 ,
- DATABASE CHEMABS [Online] CHEMICAL ABSTRACTS SERVICE, COLUMBUS, OHIO, US PINCUS, SETH H. ET AL: "In vitro effects of anti-HIV immunotoxins directed against multiple epitopes on HIV type 1 envelope glycoprotein 160" retrieved from STN Database accession no. 125:185279 XP002125550 & AIDS RES. HUM. RETROVIRUSES (1996), 12(11), 1041-1051 ,
- LEE H. PAI ET AL.: "Anti-tumor activities of immunotoxins made of monoclonal antibody B3 and various forms of Pseudomonas exotoxin" PROCEEDINGS OF THE NATIONAL ACADEMY OF SCIENCES OF USA, vol. 88, April 1998 (1998-04), pages 3358-3362, XP002125579 WASHINGTON US cited in the application
- DATABASE CHEMABS [Online] CHEMICAL ABSTRACTS SERVICE, COLUMBUS, OHIO, US MATSUSHITA, SHUZO ET AL: "Selective killing of HIV-infected cells by anti-gp120 immunotoxins" retrieved from STN Database accession no. 112:151331 XP002125551 & AIDS RES. HUM. RETROVIRUSES (1990), 6(2), 193-203 ,

## Description

### FIELD OF THE INVENTION

This invention relates to the field of immunotoxins. In particular this invention pertains to the use of immunotoxins directed against the HIV-1 gp 120 coat protein in the treatment of HIV.

### BACKGROUND OF THE INVENTION

Since the initial isolation of HIV in 1983 and its identification as the causative agent of AIDS (Barre-Sinoussi et al. (1983) Science 220: 868-871; Popovic et al. (1984) Science, 224: 497-500), tremendous efforts have been made to understand the cause and pathogenesis of AIDS, but an effective therapy leading to the cure of this disease is still in the future. To date, there are several therapeutic drugs available to treat infected patients, and these lead to prolongation of life and control of symptoms. The major approaches for the treatment of individuals with AIDS or HIV infections are the administration of drugs such as a reverse transcriptase inhibitor AZT (3'-azido-3'-deoxythymidine) or ddI (2', 3-dideoxyinosine) which act by inhibiting synthesis of proviral genome after the virion has entered the host cell, and protease inhibitors which block production of infectious virions. Although these agents can effectively inhibit HIV spread *in vitro* and *in vivo,* they do not kill those cells that are already infected. Recently a highly active antiretroviral therapy (HAART) showed encouraging results on reduction of viral loads in lymphoid tissues of HIV infected patients (Perelson et al. (1997) Nature 387: 188-191; Cavert et al. (1997) Science 276: 96-964). In this approach a cocktail consisting of a HIV protease inhibitor and two RIs (reverse transcriptase inhibitors) is administered for the treatment of HIV infected patients. Although significant progress has been made recently in the treatment of HIV-1 infection, we are not yet close to a cure for AIDS.

Immunotoxins are potent cell killing agents composed of either antibodies or antibody fragments attached to toxins (*e.g*., to protein toxins made by bacteria or plants (Pastan et al. (1995) Ann. N. Y. Acad. Sci. 758: 345-354; Thrush (1996) Ann. Rev. Immunol. 14: 49-71)). Ricin, diphtheria toxin and *Pseudomonas* exotoxin A have been widely used for purpose. The development of immunotoxins for the therapy of cancer, autoimmune diseases and other immunological disorders has been ongoing for the past two decades.

A number of immunotoxins have been made with *Pseudomonas* exotoxin A (PE). Using recombinant DNA technology the cell-binding domain of PE has been deleted along with another nonessential portion to generate a molecule (PE38) which retains its cell killing activity when targeted to cells by ligands, antibodies, or antibody fragments. Using, this approach, several recombinant immunotoxins directed at antigens on cancer cells have been constructed that are capable of curing tumors growing in nude mice. Several of these are currently undergoing clinical trials in leukemias and in colon and breast cancers and have produced significant tumor regression (Pastan et al. (1995) Ann. N. Y. Acad. Sci. 758: 345-354; Pai et al. (1996) Nature Med- 2:3 50-353).

Despite the successes obtained using immunotoxins in the treatment of cancer, immunotoxins, and in particular immunotoxins utilizing a *Pseudomonas* exotoxin have not been well studied for the treatment of HIV.

### SUMMARY OF THE INVENTION

This invention provides an immunotoxin comprising a cytotoxin attached to an anti-gp120 antibody having the binding specificity of 3B3 and a minimum binding affinity of 3B3, wherein the immunotoxin specifically binds to and kills mammalian cells infected with HIV-1. The antibody 3B3 has a high affinity and a broad cross-reactivity with many laboratory and clinical isolates of HIV. The cytotoxic component of the immunotoxin can be virtually an cytotoxin including, but not limited to ricin, abrin, a modified diphtheria toxin (*e.g.* DT388), and a modified *Pseudomonas* exotoxin (*e.g*. PE38). Particularly preferred immunotoxins comprise a modified *Pseudomonas* exotoxin (*e.g*., PE38, PE40, PE38KDEL, PE38REDL, *etc.*) with an immunotoxin comprising PE38 being most preferred. The immunotoxin can include virtually any 3B3 antibody, however particularly preferred antibodies include a single-chain Fv (scFv), a single-chain Fab (scFab), and a disulfide stabilized Fv (dsFv). The antibody can comprise a recombinantly expressed single-chain Fv. In a most preferred embodiment, the antibody is 3B3(Fv). The 3B3 antibody and the cytotoxin can be chemically conjugated together or they can be a fusion protein. In the latter case the immunotoxin can be recombinantly expressed (*i.e*. a recombinantly expressed fusion protein). In a most preferred embodiment, the immunotoxin is 3B3(Fv)-PE38. Any of the immunotoxins described herein can be suspended or dissolved in a pharmaceutically acceptable carrier or excipient.

In another embodiment, this invention provides for nucleic acids (*e.g*. DNA or RNA) that encodes all or part of any of the immunotoxins described herein. When the nucleic acid encodes a part of the immunotoxin, the nucleic acid encodes at least a cytotoxin in fusion with at least a portion (*e.g.* V_{H}, V_{L}, CDR, *etc.*) of a 3B3 antibody. In a preferred embodiment, the nucleic acid comprises a nucleic acid sequence that encodes a single-chain antibody having the binding specificity of 3B3; and a nucleic acid sequence that encodes a modified *Pseudomonas* exotoxin.

This invention also provides methods of killing a cell displaying a gp120 protein or a fragment thereof (e.g. a CD4 binding domain). The methods involve contacting the cell ex vivo with an immunotoxin described herein. In a particularly preferred embodiment, the methods involve contacting the cell with a 3B3(Fv)-PE38 immunotoxin.

The invention also comprises an immunotoxin comprising a modified *Pseudomonas* exotoxin attached to an anti-gp120 antibody having the binding specificity of 3B3 and minimum affinity of 3B3, for use in a method of killing or inhibiting the growth of cells bearing gp 120 protein or fragment thereof by administering said immunotoxin to an organism containing said cells in an amount sufficient to kill or inhibit the growth of said cells, wherein said immunotoxin specifically binds to and kills mammalian cells infected with HIV-1. In a preferred embodiment, the immunotoxin is a 3B3(Fv) attached to a modified *Pseudomonas* exotoxin (e.g. 3B3(Fv)-PE38). A protease inhibitor and/or a reverse transcriptase inhibitor can additionally be administered to the organism. In still another embodiment,
both a protease inhibitor and a reverse transcriptase inhibitor are administered to the organism and then the reverse transcriptase inhibitor is with drawn while maintaining protease inhibitor dosing.

The organism can be a mammal (*e.g*. a human) infected with HIV.

This invention also provides kits for killing cells that display a gp120 protein or fragment thereof (*e.g*. a CD4 binding domain). The kits preferably comprise a container containing an immunotoxin comprising a cytotoxin attached to an anti-gp 120 antibody having the binding specificity of 3B3 (*e.g*. 3B3(Fv)) and a minimum binding affinity of 3B3 (*e.g*. 3B3(Fv)), wherein said immunotoxin specifically binds to and kills mammalian cells infected with HIV-1. The immunotoxin can be any one or more of the immunotoxins described herein and can optionally be suspended or dissolved in a pharmacologically acceptable excipient. The container may contain a unit dosage of said immunotoxin. In a particularly preferred embodiment, the cytotoxin comprising the immunotoxin is ricin, abrin, a modified diphtheria toxin (*e.g.* DT388), or a modified *Pseudomonas* exotoxin (*e.g*., PE38, PE40, PE38KDEL, PE38REDL, *etc.).* One preferred kit includes the immunotoxin 3B3(Fv)-PE38.

### DEFINITIONS

The terms "polypeptide", "peptide", or "protein" are used interchangeably herein to designate a linear series of amino acid residues connected one to the other by peptide bonds between the alpha-amino and carboxy groups of adjacent residues. The amino acid residues are preferably in the natural "L" isomeric form. However, residues in the "D" isomeric form can be substituted for any L-amino acid residue, as long as the desired functional property is retained by the polypeptide. In addition, the amino acids, in addition to the 20 "standard" amino acids, include modified and unusual amino acids, which include, but are not limited to those listed in 37 CFR ∋1.822(b)(4). Furthermore, it should be noted that a dash at the beginning or end of an amino acid residue sequence indicates either a peptide bond to a further sequence of one or more amino acid residues or a covalent bond to a carboxyl or hydroxyl end group.

The term "binding polypeptide" refers to a polypeptide that specifically binds to a target molecule (*e.g*. a cell receptor) in a manner analogous to the binding of an antibody to an antigen. Binding polypeptides are distinguished from antibodies in that binding polypeptides are not ultimately derived from immunoglobulin genes or fragments of immunoglobulin genes.

The term "conservative substitution" is used in reference to proteins or peptides to reflect amino acid substitutions that do not substantially alter the activity (specificity or binding affinity) of the molecule. Typically conservative amino acid substitutions involve substitution one amino acid for another amino acid with similar chemical properties (*e.g*. charge or hydrophobicity). The following six groups each contain amino acids that are typical conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

The term "nucleic acid" refers to deoxyribonucleotides or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g*. degenerate codon substitutions) and complementary sequences and as well as the sequence explicitly indicated.

Specifically, degenerate codon substitutions may be achieved by generating sequences in which the third position of one or more selected (or all) codons is substituted with mixed-base and/or deoxyinosine residues (Batzer et al. (1991) Nucleic Acid Res. 19: 5081; Ohtsuka et al. (1985) J. Biol. Chem. 260: 2605-2608; and Cassol *et al.* (1992); Rossolini et al., (1994) Mol. Cell. Probes 8: 91-98). The term nucleic acid is used interchangeably with gene, cDNA, and mRNA encoded by a gene.

The terms "isolated" or "biologically pure" refer to material which is substantially or essentially free from components which normally accompany it as found in its native state. However, the term "isolated" is not intended refer to the components present in an electrophoretic gel or other separation medium. An isolated component is free from such separation media and in a form ready for use in another application or already in use in the new application/milieu.

The term "residue" as used herein refers to an amino acid that is incorporated into a polypeptide. The amino acid may be a naturally occurring amino acid and, unless otherwise limited, may encompass known analogs of natural amino acids that can function in a similar manner as naturally occurring amino acids.

A "chimeric molecule" is a molecule comprising two or more molecules that exist separately in their native state joined together to form a single entity (molecule) having the desired functionality of all of its constituent molecules. The component molecules can be chemically conjugated directly or through a linker or, where all of the component molecules are polypeptides, the chimeric molecule may be a fusion protein that can be recombinantly expressed. Frequently, one of the constituent molecules of a chimeric molecule is a "targeting molecule". The targeting molecule is a molecule such as a ligand or an antibody or antibody fragment that specifically binds to its corresponding target, for example a protein displayed on a cell surface. Where the targeting molecule is an antibody or an antibody fragment and another component is a cytotoxin, the chimeric molecule can be referred to as an immunotoxin.

A "fusion protein" refers to a polypeptide formed by the joining of two or more polypeptides through a peptide bond formed between the amino terminus of one polypeptide and the carboxyl terminus of another polypeptide. The fusion protein may be formed by the chemical coupling of the constituent polypeptides or it may be expressed as a single polypeptide from nucleic acid sequence encoding the single contiguous fusion protein. A single chain fusion protein is a fusion protein having a single contiguous polypeptide backbone.

A "spacer" as used herein refers to a peptide that joins the proteins comprising a fusion protein. Generally a spacer has no specific biological activity other than to join the proteins or to preserve some minimum distance or other spatial relationship between them. However, the constituent amino acids of a spacer may be selected to influence some property of the molecule such as the folding, net charge, or hydrophobicity of the molecule.

Abbreviations used here for the twenty naturally occurring amino acids, the five naturally occurring nucleic acids and the eleven nucleic acid degeneracies (wobbles) follow conventional usage. In the polypeptide notation used herein, the left-hand direction is the amino terminal direction and the right-hand direction is the carboxy-terminal direction. In the nucleic acid notation used herein, the left-hand direction is the 5' direction and the right-hand direction is the 3' direction.

The terms "isolated" or "substantially purified", when referring to recombinantly produced proteins, means a chemical composition which is essentially free of other cellular components. Such a composition is preferably in a homogeneous state although it can be in either a dry or aqueous solution. Purity and homogeneity are typically determined using analytical chemistry techniques such as polyacrylamide gel electrophoresis or high performance liquid chromatography. A protein which is the predominant species present in a preparation is substantially purified. Generally, a substantially purified or isolated protein will comprise more than 80% of all macromolecular species present in the preparation. Preferably, the protein is purified to represent greater than 90% of all macromolecular species present. More preferably the protein is purified to greater than 95%, and most preferably the protein is purified to essential homogeneity, wherein other macromolecular species are not detected by conventional techniques.

The term "labeled antibody" as used herein refers to an antibody bound to a label such that detection of the presence of the label (*e.g*. as bound to a biological sample) indicates the presence of the antibody.

Cytotoxin refers to a molecule that when contacted with a cell brings about the death of that cell.

The phrase "binding specificity", "specifically binds to an antibody" or "specifically immunoreactive with," when referring to a protein or carbohydrate, refers to a binding reaction which is determinative of the presence of the protein or carbohydrate in the presence of a heterogeneous population of proteins and other biologics. Thus, under designated immunoassay conditions, the specified antibodies bind to a particular protein or carbohydrate and do not bind in a significant amount to other proteins or carbohydrates present in the sample. Specific binding to an antibody under such conditions may require an antibody that is selected for its specificity for a particular protein or carbohydrate. For example, antibodies raised to the gp120 protein antigens may be selected to provide antibodies that are specifically immunoreactive with gp120 proteins and not with other proteins. A variety of immunoassay formats may be used to select antibodies specifically immunoreactive with a particular protein or carbohydrate. For example, solid-phase ELISA immunoassays are routinely used to select antibodies specifically immunoreactive with a protein or carbohydrate. See Harlow and Lane (1988) Antibodies, A Laboratory Manual, Cold Spring Harbor Publications, New York, for a description of immunoassay formats and conditions that can be used to determine specific immunoreactivity.

The terms "recombinant DNA," "recombinant nucleic acid" or "recombinantly produced DNA" refer to DNA which has been isolated from its native or endogenous source and modified either chemically or enzymatically by adding, deleting or altering naturally-occurring flanking or internal nucleotides. Flanking nucleotides are those nucleotides which are either upstream or downstream from the described sequence or sub-sequence of nucleotides, while internal nucleotides are those nucleotides which occur within the described sequence or subsequence.

The terms "recombinant protein" or "recombinantly produced protein" or "recombinantly expressed protein" refer to a peptide or protein produced using non-native cells that do not have an endogenous copy of DNA able to express the protein. The cells produce the protein because they have been genetically altered by the introduction of the appropriate nucleic acid sequence. The recombinant protein will not be found in association with proteins and other subcellular components normally associated with the cells producing the protein.

Mutations in proteins are designated by nomenclature consisting of the peptide sequence in which the mutation occurs, a representation of the non-mutated amino acid, followed by its position, followed by the representation of the mutated amino acid. Thus, for example, a mutation designated B3(Fv)V_{L} S7T is a mutation from serine (S) to threonine (T) at position 7 of the V_{L} chain of B3(Fv).

The term "*Pseudomonas* exotoxin" (PE) as used herein refers to a full-length native (naturally occurring) PE or a PE that has been modified. The full length native sequence of *Pseudomonas* exotoxin can be found in Gray et al. (1984) Proc. Natl. Acad Sci. USA, 81: 2645-2649 (*see also* U.S. Patent 5,602,095). A "modified *Pseudomonas* exotoxin" refers to a *Pseudomonas* exotoxin that has an amino acid sequence different than the amino acid sequence of the native *Pseudomonas* exotoxin. Such modifications may include, but are not limited to, elimination of domain Ia, various amino acid deletions in domains II and III, single amino acid substitutions (*e.g*., replacing Lys with Gln at positions 590 and 606), and the addition of one or more sequences at the carboxyl terminus such as KDEL (SEQ ID No:9) and REDL (SEQ ID No:10) (*see* Siegall et al., (1989) J. Biol. Chem. 264: 14256-14261). Thus, for example, PE38 refers to a truncated *Pseudomonas* exotoxin composed of amino acids 253-364 and 381-613 *(see* commonly assigned U.S. Patent Application Serial Number 07/901,709 filed June 18,1992). The native C-terminus of PE,REDLK (SEQ ID No:11) (residues 609-613), may be replaced with sequences such as KDEL (SEQ ID No:9) and REDL (SEQ ID No:10). Lys⁵⁹⁰ and Lys⁶⁰⁶ may be each mutated to Gln.(see commonly assigned U.S. Patent Application Serial Number 07/522,563 filed May 14, 1990).

As used herein, an "antibody" refers to a protein consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

A typical immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist as intact immunoglobulins or as a number of well characterized fragments produced by digestion with various peptidases. Thus, for example, pepsin digests an antibody below the disulfide linkages in the hinge region to produce F(ab)'2, a dimer of Fab which itself is a light chain joined to V_{H}-C_{H}1 by a disulfide bond. The F(ab)'2 may be reduced under mild conditions to break the disulfide linkage in the hinge region thereby converting the (Fab')2 dimer into an Fab' monomer. The Fab' monomer is essentially a Fab with part of the hinge region *(see,* Paul (1993) Fundamental Immunology, Raven Press, N.Y., for a more detailed description of other antibody fragments). While various antibody fragments are defined in terms of the digestion of an intact antibody, one of skill will appreciate that such Fab' fragments may be synthesized de novo either chemically or by utilizing recombinant DNA methodology. Thus, the term antibody, as used herein also includes antibody fragments either produced by the modification of whole antibodies or synthesized de novo using recombinant DNA methodologies. Preferred antibodies include single chain antibodies (antibodies that exist as a single polypeptide chain), more preferably single chain Fv antibodies (sFv or scFv) in which a variable heavy and a variable light chain are joined together (directly or through a peptide linker) to form a continuous polypeptide. The single chain Fv antibody is a covalently linked V_{H}-V_{L} heterodimer that may be expressed from a nucleic acid including V_{H}- and V_{L}-encoding sequences either joined directly or joined by a peptide-encoding linker (Huston, et al. (1988) Proc. Nat. Acad. Sci. USA, 85: 5879-5883). While the V_{H} and V_{L} are connected to each as a single polypeptide chain, the V_{H} and V_{L} domains associate non-covalently.

A 3B3 antibody refers to an antibody having the binding specificity of 3B3(Fv) exemplified herein(see SEQ ID NO:1). Particularly preferred 3B3 antibodies have a binding affinity for the gp120 (*e.g*. gp120 CD4 binding domain) comparable to or greater than the binding affinity of Ara 3B3 Fab or 3B3(scFv)-PE38 exemplified herein *(see* Table
1). In a preferred embodiment, 3B3 antibodies have a binding affinity (K_{D}) greater (lower K_{D}) than about 3.0 x 10⁻⁸, preferably greater than about 3.6 x 10⁻⁸, more preferably greater than about 3.7 x 10⁻⁸, and most preferably greater than about 3.0 x 10⁻⁹ as determined using a BIAcore assay as described herein in Example 1. 3B3 antibodies include modified variants of the 3B3(Fv) exemplified herein. Such modifications include, but are not limited to, conservative amino acid substitutions, mutants derived by CDR walking as described herein, chain shuffling variants, and so forth.

HIV is a family of viruses, *e.g.*, HIV-1 and HIV-2, well known to those of skill in the art. The original isolates of these viruses were variably referred to as lymphadenopathy virus (LAV, Barre-Sinoussi et al. (1983) Science 220:868-871), human T-cell lymphotropic virus III (HTLV-III, Popovic et al. (1984) Science 224:497) and AIDS-associated retrovirus (ARV, Levy et al. (1984) Science 225 840-842). These isolates were originally termed "human T-cell lymphotropic retrovirus (hTLR)". Subsequently, the name HIV has been given to these retroviruses by an international committee. Thus, HIV (and particularly HIV-1) shall be used herein as an equivalent to hTLR. Examples of HIV-1 were previously called LAV, ARV and HTLV-III. Among the identifying characteristics of HIV retroviruses are (i) being an etiologic of AIDS, (ii) being cytopathic *in vitro,* (iii) having a tropism for CD4-bearing cells, and (iv) having elements trans-activating the expression of viral genes acting at the LTR level.

The term "HIV-1 protein" is used herein to refer to a protein (p), glycoprotein (gp), or fragment thereof that is characteristically found in, and therefore characteristic of HIV-1. Typical HIV-1 proteins include, but are not limited to gp160, gp120, p65, p55, p51, gp41, p31, p24 and p18 (number refers to apparent molecular weight in kilodaltons).

### BRIEF DESCRIPTION OF THE DRAWINGS

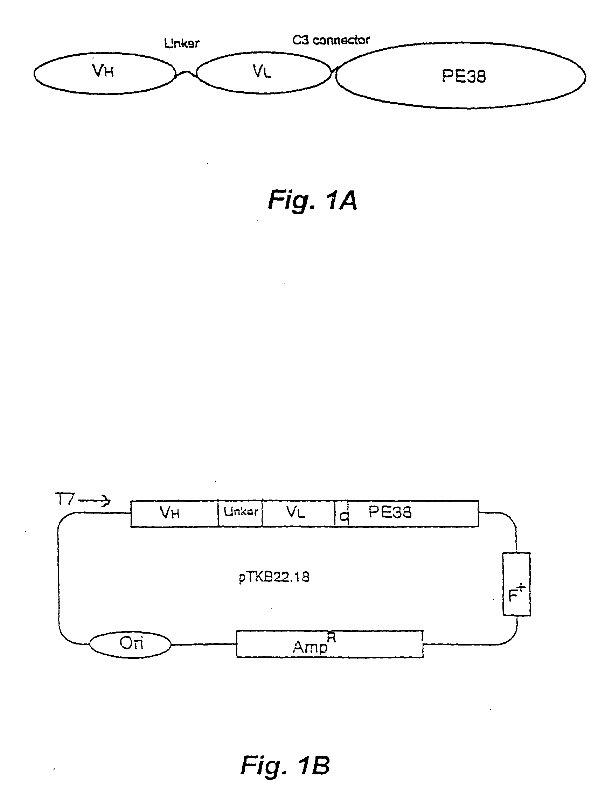

Figures 1A and 1B illustrate construction of the plasmid for expression of 3B3(Fv)-PE38 immunotoxin. Figure 1A shows a schematic of of3B3(Fv)-PE38. The V_{H} and V_{L} portion of antibody 3B3 are linked by a fifteen amino acid linker and fused to the translocation and ADP ribosylation domains of PE. Figure 1B shows the expression plasmid pTKB22.18 that encodes the V_{H} and V_{L} domains of antibody 3B3 fused in frame to PE38. The carboxy terminus of V_{H} is linked to the amino terminus of the toxin molecule (PE38).

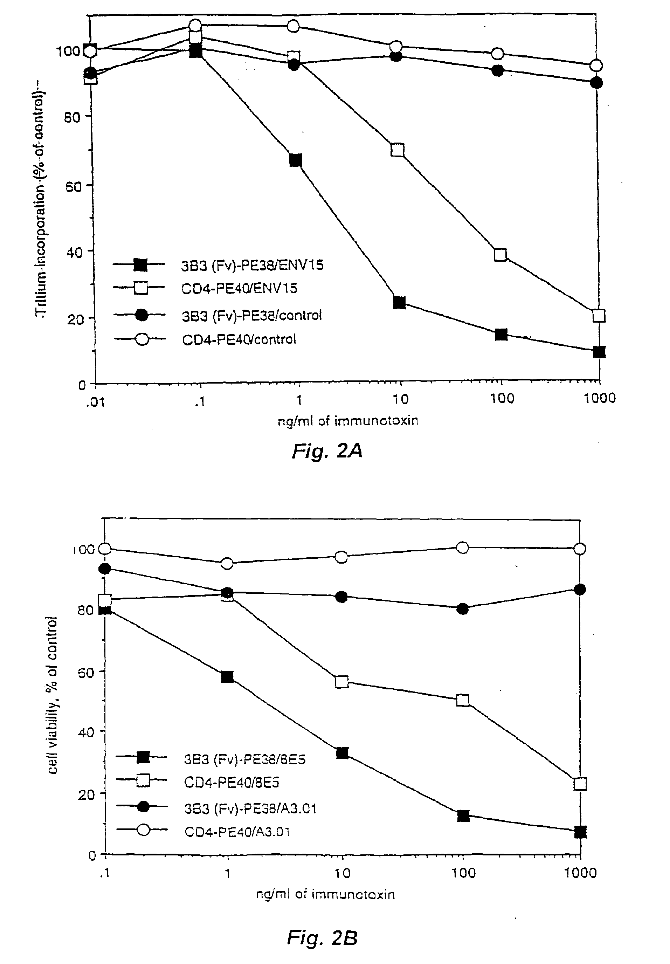

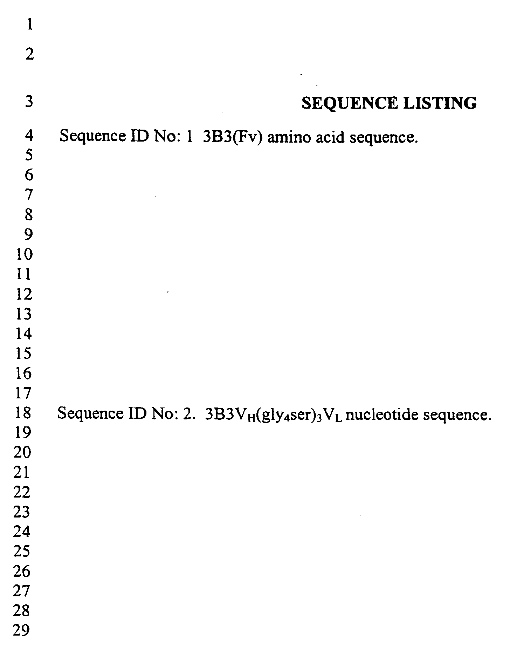

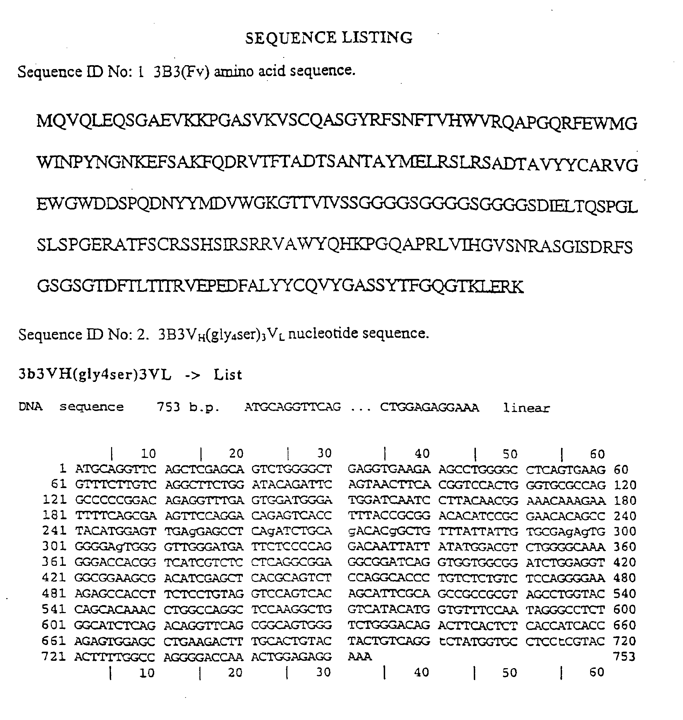

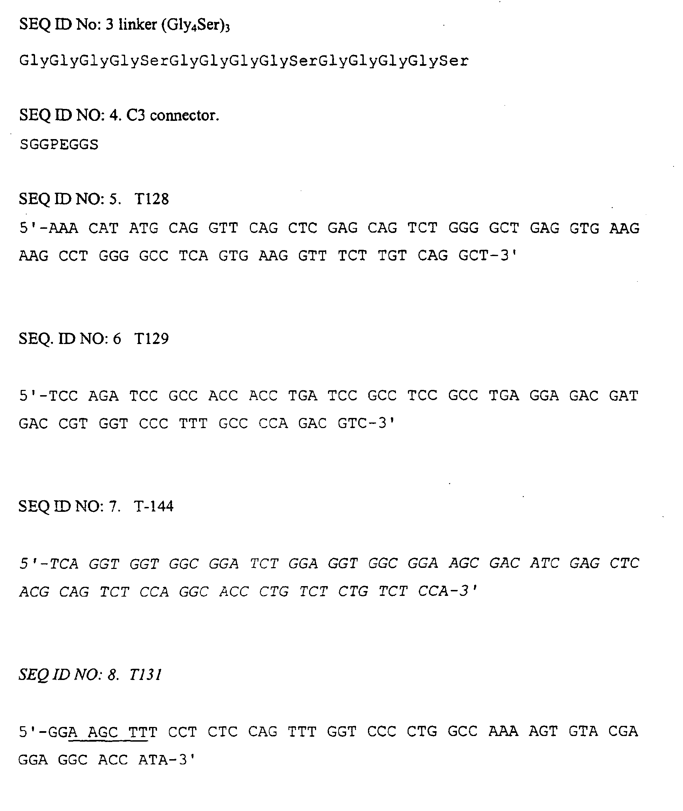

Figures 2A and 2B illustrate specific cytotoxicity of CD4-PE40 and 3B3(Fv)-PE38 immunotoxin towards a gp120 expressing cell line (Figure 1A) and a HIV-1 chronically infected lymphocyte cell line. In figure 2A, the squares represent ENV15, a gp120 expressing CHO cell line; and circles represent a control CHO cell line. In Figure 2B the squares, represent 8E5, a lymphocyte cell line which is chronically infected with HIV-1 virus; and circles represent a control parental cell line A3.01. Cell viability assays were performed as described herein.

### DETAILED DESCRIPTION

### I. Treatment of HIV using gp120-directed immunotoxins.

This invention provides a chimeric cytotoxin molecule that uses, as a targeting moiety, a 3B3 antibody (preferably a 3B3 Fv fragment), that has a high affinity and a broad cross-reactivity with many laboratory and clinical isolates of HIV. The 3B3 antibody is attached to a cytotoxic moiety (*e.g.* a *Pseudomonas* exotoxin) and is capable of specifically targeting and killing cells that display an HIV gp120 coat protein.

The gp120 coat protein is characteristically displayed on the surface of HIV-infected cells. Moreover, the gp120 protein is displayed on cells that are not dividing and in which HIV is not rapidly propagating. Thus, the chimeric cytotoxins of this invention are capable of targeting and killing cells (*e.g*. dendritic cells of lymph nodes) that act as quiescent reservoirs of HIV. By specifically attacking and killing cells that act as HIV reservoirs, the immunotoxins of this invention augment the activities of reverse transcriptase inhibitors and protease inhibitors in purging the organism of HIV.

The parental antibody from which 3B3 was derived, was isolated from a combinatorial phage display library constructed from bone marrow RNA of an infected individual (Burton et al. (1991) Proc. Natl. Acad. Sci. USA, 88: 10134-10137). While there is a high degree of inter-isolate sequence variability of gp120, the 3B3 antibodies of this invention reacts with the conserved CD4-binding site of gp120, the external subunit of the envelope glycoprotein (Barbas et al. (1991) Proc. Natl. Acad. Sci. USA, 91: 3809-3813). Moreover, unlike other monoclonal antibodies that are also directed to CD4 binding epitope, the parent of the 3B3 antibody can neutralize many different laboratory strains of HIV-1 as well as many primary isolates (Kessler et al. (1997) Hum. Retrovinises 13: 575-582; Burton et al. (1994) Science 266: 1024-1027; Trkola et al. (1995) J. Virol., 69: 6609-6617).

In a particularly preferred embodiment, the chimeric molecules of this invention utilize a single-chain 3B3 attached to a cytotoxic polypeptide (*e.g*. modified *Pseudomonas* exotoxin) and preferably expressed as a fusion protein. The preferred immunotoxin 3B3(Fv)-PE38 specifically kills HIV-infected lymphocytes without affecting cells that do not display gp120. It is believed the immunotoxin will be tolerated at significantly higher doses than a CD4-directed immunotoxin and will also show significantly higher specific toxicity to cells displaying a gp120 protein than CD4-directed immunotoxins.

### II. Other uses of gp120-Directed immunotoxins.

The immunotoxins of this invention have numerous uses other than the treatment of HIV. For example, in one embodiment, the immunotoxins of this invention can be used *ex vivo* to reduce and/or eliminate the HIV viral load of cells, tissues, or organs derived from HIV-infected organisms. This will be of use in reducing or eliminating HIV-infected cells in culture, *e.g.*, either where the cells are simply going to be propagated or maintained or prior to re-infusion back into the donor (*e.g*. to generate a population of uninfected stem or precursor cells). The immunotoxins can also be used in establishing transformed cell lines derived from HIV-infected sources or in providing cells, tissues, or organs for transplant where there is no compatible donor other than an HIV-infected organism (*e.g*. human).

The immunotoxins of this invention can also be used for detecting the presence or absence and/or quantifying the number of infected cells. In this embodiment, a cell culture is treated with an immunotoxin of this invention and the resulting reduction of cell number (*i.e*., due to cell killing by the immunotoxin) is assayed (*e.g*., by comparison to a similar untreated culture). A significant reduction in cell number caused by the immunotoxin, *e.g*. in comparison to appropriate controls, will indicate the presence and/or quantity of infected cells. This assay may be of particular use in evaluating viral load in subjects where virus is essentially undetectable in blood and exists primarily in quiescent reservoirs.

### III. 3B3 Antibodies.

The antibodies used in this invention specifically bind to the HIV gp120 coat protein. In particular the antibodies are selected that bind to the conserved CD4-binding site of gp120, the external subunit of the envelope glycoprotein (Barbas et al. (1994) Proc. Natl. Acad. Sci. USA, 91: 3809-3813). Particularly preferred antibodies are derived from the antibody 3B3 (which is a Fab fragment of whole antibody IgG1b12 (Burton et al. (1994) Science, 266: 1024-1027) which itself is derived from Fab b12 described in U.S. Patent 5,652,138) that has been affinity enhanced by complementarity-determining region (CDR) walking (*see,* Barbas et al. (1991) Proc. Natl. Acad. Sci. USA. 91: 3809-3813). The amino acid and nucleic acid sequences of 3B3 are provided in SEQ ID NOS:1 and 2, respectively) while the amino acid and nucleic acid sequences of b12 are provided in U.S. Patent 5,652,138.

### A) Modification of 3B3 to produce other 3B3 antibodies.

Using the 3B3 sequence information provided herein the 3B3 antibody can readily be expressed in fusion with virtually any polypeptide, in particular with polypeptide cytotoxins (*e.g. Pseudomonas* exotoxin, diphtheria toxin, *etc.*). The 3B3 antibodies of this invention can be routinely modified as long as the binding affinity and specificity of 3B3 is retained.

Means of modifying antibodies and screening for affinity and specificity are well known to those of skill in the art. Such modifications include, but are not limited to, site-directed mutagenesis, and complementarity-determining region (CDR) walking.

Site-directed mutagenesis can be used to specifically alter resides believed to effect binding (*e.g*. as determined through modeling of the antibody/substrate interaction).

In a particularly preferred embodiment, the 3B3 antibodies are modified by CDR walking. In this approach, complementarity determining regions (CDRs) are targeted for random mutagenesis. They are then selected for increased binding affinity ,*e.g*, by the phage-display approach. The improvement of the Fab b12 antibody using this approach is described in detail by Barbas et al. (1994) Proc. Natl. Acad. Sci. USA, 91: 3809-3813.

In still another embodiment, the 3B3 antibody can be modified so that it is a disulfide-stabilized antibody. Disulfide-stabilized 3B3 antibodies include at least two different polypeptides that are joined together by a linker, most preferably by a disulfide linkage (*e.g*. formed between respective cysteines in each chain). 3B3 antibodies comprising two polypeptide chains joined by a disulfide linkage have a reduced tendency to aggregate, show a generally longer serum half-life and are said to be "stabilized". Thus a disulfide-stabilized 3B3 antibody, as used herein, refers to a 3B3 antibody comprising at least two polypeptides joined by at least one disulfide linkage. The disulfide linkage, however, need not be the only linkage joining the polypeptides. Thus, for example, a variable light and variable heavy chain of an antibody may be joined by a disulfide linkage and additionally joined by terminal peptide linker. Such a molecule may thus be expressed as a single chain fusion protein (*e.g*. V_{H-}-peptide-V_{L}) where the V_{H} and V_{L} polypeptides are subsequently cross-linked by the formation of a disulfide linkage. Methods of producing disulfide-stabilized binding agents can be found in U.S. Patent 5,747,654.

### B) Screening for 3B3 antibodies.

As indicated above 3B3 antibodies are antibodies that have the binding specificity of 3B3 or 3B3(Fv) and a binding affinity about equal to or greater than 3B3. Antibodies having the specificity of 3B3 or 3B3(Fv) can be identified by their ability to cross react (bind to) either the gp 120 epitope bound by 3B3 or with anti-idiotypic antibodies raised against 3B3 or 3B3(Fv). In addition, the 3B3 antibody will preferably not bind to epitopes not bound by 3B3 or 3B3(Fv).

### 1) Cross-reactivity with anti-idiotypic antibodies.

The idiotype represents the highly variable antigen-binding site of an antibody and is itself immunogenic. During the generation of an antibody-mediated immune response, an individual will develop antibodies to the antigen as well as anti-idiotype antibodies, whose immunogenic binding site (idiotype) mimics the antigen.

3B3-derived antibodies can then be recognized by their ability to specifically bind to the 3B3- anti-idiotypic antibodies.

Anti-idiotypic antibodies can be raised against the variable regions of 3B3 using standard methods well known to those of skill in the art. Briefly, anti-idiotype antibodies can be made by injecting 3B3 antibodies or fragments thereof (*e.g*., CDRs) into an animal thereby eliciting antiserum against various antigenic determinants on the antibody, including determinants in the idiotypic region.

Methods for the production of anti-analyte antibodies are well known in the art. Large molecular weight antigens (greater than approx. 5000 Daltons) can be injected directly into animals, whereas small molecular weight compounds (less than approx. 5000 Daltons) are preferably coupled to a high molecular weight immunogenic carrier, usually a protein, to render them immunogenic. The antibodies produced in response to immunization can be utilized as serum, ascites fluid, an immunoglobulin (Ig) fraction, an IgG fraction, or as affinity-purified monospecific material.

Polyclonal anti-idiotype antibodies can be prepared by immunizing an animal with the antibodies of this invention prepared as described above. In general, it is desirable to immunize an animal that is species and allotype-matched with the animal from which the antibody (*e.g*. phage-display library) was derived. This minimizes the production of antibodies directed against non-idiotypic determinants. The antiserum so obtained is then usually absorbed extensively against normal serum from the same species from which the phage-display library was derived, thereby eliminating antibodies directed against non-idiotypic determinants. Absorption can be accomplished by passing antiserum over a gel formed by crosslinking normal (nonimmune) serum proteins with glutaraldehyde. Antibodies with anti-idiotypic specificity will pass directly through the gel, while those having specificity for non-idiotypic determinants will bind to the gel. Immobilizing nonimmune serum proteins on an insoluble polysaccharide support (*e.g*., sepharose) also provides a suitable matrix for absorption.

Monoclonal anti-idiotype antibodies can be produced using the method of Kohler et al. (1975) Nature 256: 495. In particular, monoclonal anti-idiotype antibodies can be prepared using hybridoma technology which comprises fusing (1)spleen cells from a mouse immunized with the antigen or hapten-carrier conjugate of interest (*i.e*., the antibodies or this invention or subsequences thereof) to (2) a mouse myeloma cell line which has been selected for resistance to a drug (*e.g*., 8-azaguanine). In general, it is desirable to use a myeloma cell line that does not secrete an immunoglobulin. Several such lines are known in the art. A preferred cell line is P3X63Ag8.653. This cell line is on deposit at the American Type Culture Collection as CRL-1580.

Fusion can be carried out in the presence of polyethylene glycol according to established methods (*see, e.g.,* Monoclonal Antibodies, R. Kennett, J. McKearn & K. Bechtol, eds. N.Y., Plenum Press, 1980, and Current Topics in Microbiology & Immunology, Vol. 81, F. Melchers, M. Potter & N. L. Warner, eds., N.Y., Springer-Verlag, 1978). The resultant mixture of fused and unfused cells is plated out in hypoxanthine-aminopterin-thymidine (HAT) selective medium. Under these conditions, only hybrid cells will grow.

When sufficient cell growth has occurred, (typically 10-14 days post-fusion), the culture medium is harvested and screened for the presence of monoclonal idiotypic, anti-analyte antibody by any one of a number of methods which include solid phase RIA and enzyme-linked immunosorbent assay. Cells from culture wells containing antibody of the desired specificity are then expanded and recloned. Cells from those cultures which remain positive for the antibody of interest are then usually passed as ascites tumors in susceptible, histocompatible, pristane-primed mice.

Ascites fluid is harvested by tapping the peritoneal cavity, retested for antibody, and purified as described above. If a nonsecreting myeloma line is used in the fusion, affinity purification of the monoclonal antibody is not usually necessary since the antibody is already homogeneous with respect to its antigen-binding characteristics. All that is necessary is to isolate it from contaminating proteins in ascites, *i.e*., to produce an immunoglobulin fraction.

Alternatively, the hybrid cell lines of interest can be grown in serum-free tissue culture and the antibody harvested from the culture medium. In general, this is a less desirable method of obtaining large quantities of antibody because the yield is low. It is also possible to pass the cells intravenously in mice and to harvest the antibody from serum. This method is generally not preferred because of the small quantity of serum which can be obtained per bleed and because of the need for extensive purification from other serum components. However, some hybridomas will not grow as ascites tumors and therefore one of these alternative methods of obtaining antibody must be used.

### 2) Cross-reactivity with the 3B3 gp120 epitope.

Instead of the anti-idiotypic antibody, putative 3B3 antibodies can be identified by cross-reactivity with 3B3 or 3B3(Fv), against the gp120 epitope bound by 3B3.

This can be ascertained by providing cells expressing native or recombinant gp120 or by providing the isolated gp120 attached to a solid support. Competition between the putative 3B3 antibody and the 3B3(Fv) of SEQ ID NO: 1, *e.g*. in an epitope-mapping format establishes that the antibodies are competing for the same epitope. The putative antibodies are then screened as described below.

### 3) Cross-reactivity measurements.

Immunoassays in the competitive binding format are preferably used for crossreactivity determinations. For example, the 3B3 gp120 epitope or 3B3 anti-idiotypic antibody is immobilized to a solid support. The putative 3B3 derived antibodies (*e.g*. generated by selection from a phage-display library or modification of 3B3) added to the assay compete with the 3B3 antibodies of SEQ ID NO:1, respectively binding to the immobilized epitope or anti-idiotypic antibody. The ability of the putative 3B3-derived antibodies to compete with the binding of the 3B3 antibody (SEQ ID NO:1) to the immobilized protein are compared. The percent crossreactivity of the proteins is calculated, using standard calculations.

If the putative 3B3-derived antibody competes with 3B3 or 3B3(Fv) and has a binding affinity comparable to or greater than 3B3 with the same target then the putative 3B3 antibody is regarded as a 3B3 (derived) antibody.

### IV. Modified Pseudomonas exotoxin.

*Pseudomonas* exotoxin A (PE) is an extremely active monomeric protein (molecular weight 66 kD), secreted by *Pseudomonas aeruginosa,* that inhibits protein synthesis in eukaryotic cells through the inactivation of elongation factor 2 (EF-2) by catalyzing its ADP-ribosylation (catalyzing the transfer of the ADP ribosyl moiety of oxidized NAD onto EF-2).

The toxin contains three structural domains that act in concert to cause cytotoxicity. Domain Ia (amino acids 1-252) mediates cell binding. Domain II (amino acids 253-364) is responsible for translocation into the cytosol and domain III (amino acids 400-613) mediates ADP ribosylation of elongation factor 2, which inactivates protein synthesis and causes cell death. The function of domain Ib (amino acids 365-399) remains undefined, although a large part of it, amino acids 365-380, can be deleted without loss of cytotoxicity (*see* Siegall et al., J. Biol. Chem. 264: 14256-14261 (1989).

When a targeting molecule (*e.g*. a 3B3 antibody) is to be attached to the PE, the PE is preferably one in which Ia (amino acids 1 through 252) substantially or completely. deleted and amino acids 365 to 380 have been deleted from domain Ib. However all of domain Ib and a portion of domain II (amino acids 350 to 394) can be deleted, particularly if the deleted sequences are replaced with a linking peptide such as GGGGS (SEQ ID No:12)

In addition, the PE molecules can be further modified using site-directed mutagenesis or other techniques known in the art, to alter the molecule for a particular desired application. Means to alter the PE molecule in a manner that does not substantially affect the functional advantages provided by the PE molecules described here can also be used and such resulting molecules are intended to be covered herein.

For maximum cytotoxic properties of a preferred PE molecule, several modifications to the molecule are recommended. An appropriate carboxyl terminal sequence to the recombinant molecule is preferred to translocate the molecule into the cytosol of target cells. Amino acid sequences which have been found to be effective include, REDLK (SEQ ID No: 11) (as in native PE), REDL (SEQ ID No:10), RDEL (SEQ ID No:13), or KDEL (SEQ ID No:9), repeats of those, or other sequences that function to maintain or recycle proteins into the endoplasmic reticulum, referred to here as "endoplasmic retention sequences" (*see, e.g.,* Chaudhary et al. Proc. Natl. Acad. Sci. USA 87:308-312 and Seetharam et al. (1991) J. Biol. Chem. 266: 17376-17381).

Deletions of amino acids 365-380 of domain Ib can be made without loss of activity. Further, a substitution of methionine at amino acid position 280 in place of glycine to allow the synthesis of the protein to begin and of serine at amino acid position 287 in place of cysteine to prevent formation of improper disulfide bonds is beneficial.

In a preferred embodiment, the targeting molecule is inserted in replacement for domain Ia. A similar insertion has been accomplished in what is known as the TGFα-PE40 molecule (also referred to as TP40) described in Heimbrook et al. (1990) Proc. Natl. Acad. Sci., USA, 87: 4697-4701 and in U.S. Patent 5,458,878.

Preferred forms of PE contain amino acids 253-364 and 381-608, and are followed by the native sequences REDLK (SEQ ID No:11) or the mutant sequences KDEL (SEQ ID No:9) or RDEL (SEQ ID No:13) Lysines at positions 590 and 606 may or may not be mutated to glutamine.

In a particularly preferred embodiment, the cytotoxin is PE38. PE38 refers to a truncated *Pseudomonas* exotoxin composed of amino acids 253-364 and 381-613 *(see* commonly assigned U.S. Patent Application Serial Number 07/901,709 filed June 18,1992). The native C-terminus of PE, REDLK (SEQ ID No:11) (residues 609-613), may be replaced with sequences such as KDEL (SEQ ID No:9) and REDL (SEQ ID No:10). Lys⁵⁹⁰ and Lys⁶⁰⁶ may be each mutated to Gln (see commonly assigned U.S. Patent Application Serial Number 07/522,563 filed May 14, 1990)

Another preferred modified Pseudomonas exotoxin is PE38QQR. This PE molecule is a truncated form ofPE composed of amino acids 253-364 and 381-608. The lysine residues at positions 509 and 606 are replaced by glutamine and at 613 are replaced by arginine (Debinski et al. (1994) Bioconj. Chem., 5: 40).

Other suitable modified *Pseudomonas* exotoxins include, but are not limited to, PE4E, a "full length" PE with a mutated and inactive native binding domain where amino acids 57, 246, 247, and 249 are all replaced by glutamates *(see, e.g.,* Chaudhary et al. (1995) J. Biol. Chem., 265: 16306), PE40 which consists of amino acids 253-613 ofPE, and PE38KDEL which lacks domain Ia (amino acids 1-252) and part of domain Ib (amino acids 365-380), and also contains an altered carboxyl terminal sequence KDEL (SEQ ID No:9) (Chaudhary et al. (1990) Proc. Natl. Acad. Sci. USA, 87: 308-12.

### V. Other cytotoxins.

While, in a preferred embodiment, the immunotoxins of this invention utilize a modified Pseudomonas exotoxin as the cytotoxic component of the chimeric molecule, other cytotoxic moieties can be used as well. Cytotoxins are well known to those of skill in the art and include, but are not limited to including ricin A chain, blocked ricin, saporin, pokeweed antiviral protein, various prodrugs, and diphtheria toxin (DT) *(see, e.g.,* Vitetta et al., Semin. (1991) Cell Biol. 2: 47-58; Tazzari et al., (1992) Br. J. Hematol. 81: 203-211; Uckun et al. (1992) Blood, 79: 2201-2214). Diphtheria toxin (DT), however, is the most frequently used cytotoxin after *Pseudomonas* exotoxin.

Like PE, diphtheria toxin (DT) kills cells by ADP-ribosylating elongation factor 2 (EF-2) thereby inhibiting protein synthesis. Diphtheria toxin, however, is divided into two chains, A and B, linked by a disulfide bridge. In contrast to PE, chain B of DT, which is on the carboxyl end, is responsible for receptor binding and chain A, which is present on the amino end, contains the enzymatic activity (Uchida et al. (1972) Science, 175: 901-903; Uchida et al. (973) J. Biol. Chem., 248: 3838-3844).

The 3B3-Diphtheria toxin fusion proteins of this invention may have the DT native receptor-binding domain removed by truncation of the Diphtheria toxin B chain. DT388, a DT in which the carboxyl terminal sequence beginning at residue 389 is removed is illustrated in Chaudhary, et al. (1991) Bioch. Biophys. Res. Comm., 180: 545- 551 (1991).

Like the PE cytotoxins, the diphtheria (DT) molecules may be attached to the 3B3 antibody by chemical conjugation or may be expressed as a fusion protein with 3B3. The genes encoding the DT protein chains may be cloned in cDNA or in genomic form by any cloning procedure known to those skilled in the art. Methods of cloning genes encoding DT fused to various ligands are also well known to those of skill in the art. See, for example, Williams et al. (1990) J. Biol. Chem. 265: 11885-11889 which describes the expression of growth-factor-DT fusion proteins.

The term "Diphtheria toxin" (DT) as used herein refers to full length native DT or to a DT that has been modified. Modifications typically include removal of the targeting domain in the B chain and, more specifically, involve truncations of the carboxyl region of the B chain.

### VI. Attachment of the 3B3 antibody to the cytotoxin.

The 3B3 antibody and the cytotoxin molecules may be joined together in any order. Thus, where the cytotoxin may be joined to either the amino or carboxy termini of the 3B3 antibody or attached to an "internal" residue. Conversely, the 3B3 antibody may also be joined to an internal region of the effector molecule. Similarly, the cytotoxin can be attached via either terminus or internal linkages.

Thus, the 3B3 antibody is preferably attached to the amino terminus of the modified *Pseudomonas* exotoxin, more preferably replacing some or all of domain Ia. alternatively the 3B3 antibody can be inserted at a point within domain III of the PE molecule. Where the 3B3 molecule is inserted within the carboxyl terminus (domain III), the 3B3 antibody is preferably fused between about amino acid positions 607 and 609 of the PE molecule. This means that the targeting molecule is inserted after about amino acid 607 of the molecule and an appropriate carboxyl end of PE is recreated by placing amino acids about 604-613 of PE after the targeting molecule. Thus, the targeting molecule is inserted within the recombinant PE molecule after about amino acid 607 and is followed by amino acids 604-613 of domain III. The targeting molecule may also be inserted into domain Ib to replace sequences not necessary for toxicity (Debinski, et al. (1991) Mol. Cell. Biol., 11: 1751-1753).

The targeting molecule and the effector molecule may be attached by any of a number of means well known to those of skill in the art. For example, the cytotoxin can be chemically conjugated, either directly or through a linker (spacer), to the 3B3 antibody.

However, in a preferred embodiment, the cytotoxin (*e.g*., PE) molecules will be fused to the targeting molecule by recombinant means. The genes encoding protein chains may be cloned in cDNA or in genomic form by any cloning procedure known to those skilled in the art *(see, e.g.,* Sambrook et al., Molecular Cloning: A Laboratory Manual, Cold Spring Harbor Laboratory, (1989)). Methods of cloning genes encoding PE fused to various ligands are well known to those of skill in the art (*see, e.g.,* Siegall et al. (1989) FASEB J., 3: 2647-2652; and Chaudhary et al. (1987) Proc. Natl. Acad. Sci. USA, 84: 4538-4542).

### A) Conjugation of the effector molecule to the targeting molecule.

In one embodiment, the targeting molecule (3B3 antibody) is chemically conjugated to the cytotoxin. Means of chemically conjugating such molecules are well known to those of skill.

The procedure for attaching an agent to an antibody will vary according to the chemical structure of the agent. Polypeptides typically contain variety of functional groups; *e.g*., carboxylic acid (COOH) or free amine (-NH₂) groups, which are available for reaction with a suitable functional group on an effector molecule to bind the effector thereto.

Alternatively, the antibody and/or cytotoxin molecule may be derivatized to expose or attach additional reactive functional groups. The derivatization may involve attachment of any of a number of linker molecules such as those available from Pierce Chemical Company, Rockford Illinois.

A "linker", as used in this context is a molecule that is used to join the targeting molecule to the effector molecule. The linker is capable of forming covalent bonds to both the targeting molecule and to the effector molecule. Suitable linkers are well known to those of skill in the art and include, but are not limited to, straight or branched-chain carbon linkers, heterocyclic carbon linkers, or peptide linkers. Where the targeting molecule and the effector molecule are polypeptides, the linkers may be joined to the constituent amino acids through their side groups (*e.g*., through a disulfide linkage to cysteine). However, in a preferred embodiment, the linkers will be joined to the alpha carbon amino and carboxyl groups of the terminal amino acids.

A bifunctional linker having one functional group reactive with a group on a particular agent, and another group reactive with an antibody, may be used to form the desired immunoconjugate. Alternatively, derivatization may involve chemical treatment of the targeting molecule, *e.g.*, glycol cleavage of the sugar moiety of a the glycoprotein antibody with periodate to generate free aldehyde groups. The free aldehyde groups on the antibody may be reacted with free amine or hydrazine groups on an agent to bind the agent thereto. (See U.S. Patent No. 4,671,958). Procedures for generation of free sulfhydryl groups on polypeptide, such as antibodies or antibody fragments, are also known (See U.S. Pat. No. 4,659,839).

Many procedure and linker molecules for attachment of various compounds including radionuclide metal chelates, toxins and drugs to proteins such as antibodies are known. See, for example, European Patent Application No. 188,256; U.S. Patent Nos. 4,671,958, 4,659,839, 4,414,148, 4,699,784; 4,680,338; 4,569,789; and 4,589,071; and Borlinghaus et al. Cancer Res. 47: 4071-4075 (1987). In particular, production of various immunotoxins is well-known within the art and can be found, for example in "Monoclonal Antibody-Toxin Conjugates: Aiming the Magic Bullet," Thorpe et al., Monoclonal Antibodies in Clinical Medicine, Academic Press, pp. 168-190 (1982), Waldmann, Science, 252: 1657 (1991), U.S. Patent Nos, 4,545,985 and 4,894,443.

In some circumstances, it is desirable to free the cytotoxin molecule from the antibody when the chimeric molecule has reached its target site. Therefore, chimeric conjugates comprising linkages which are cleavable in the vicinity of the target site may be used when the cytotoxin is to be released at the target site. Cleaving of the linkage to release the agent from the antibody may be prompted by enzymatic activity or conditions to which the immunoconjugate is subjected either inside the target cell or in the vicinity of the target site. When the target site is an HIV infected cell, a linker which is cleavable under conditions present at or within the cell (*e.g*. when exposed various proteases or acidic pH) may be used.

A number of different cleavable linkers are known to those of skill in the art. See U.S. Pat. Nos. 4,618,492; 4,542,225, and 4,625,014. The mechanisms for release of an agent from these linker groups include, for example, irradiation of a photolabile bond and acid-catalyzed hydrolysis. U.S. Pat. No. 4,671,958, for example, includes a description of immunoconjugates comprising linkers which are cleaved at the target site *in vivo* by the proteolytic enzymes of the patient's complement system. In view of the large number of methods that have been reported for attaching a variety of radiodiagnostic compounds, radiotherapeutic compounds, drugs, toxins, and other agents to antibodies one skilled in the art will be able to readily determine a suitable method for conjugating the 3B3 antibody to a suitable cytotoxin.

### B) Production of fusion proteins.

Where the 3B3-PE38 chimeric molecule is to be expressed as a fusion protein, nucleic acids are provided that encoded the 3B3 antibody and the cytotoxin. The nucleic acids are joined to provide a continuous nucleic acid that encodes both the 3B3 antibody and the cytotoxin. This nucleic acid, in an expression casssette, is transfected into an appropriate host cell that then expresses the recombinant immunotoxin. The immunotoxin is recovered, purified and refolded, if necessary to restore activity.

DNA encoding the 3B3 antibodies and/or the cytotoxins of this invention may be prepared by any suitable method, including, for example, cloning and restriction of appropriate sequences or direct chemical synthesis by methods such as the phosphotriester method of Narang et al. (1979) Meth. Enzymol. 68: 90-99; the phosphodiester method of Brown et al. (1979) Meth. Enzymol. 68: 109-151; the diethylphosphoramidite method of Beaucage et al. (1981) Tetra. Lett., 22: 1859-1862; and the solid support method of U.S. Patent No. 4,458,066.

Chemical synthesis produces a single stranded oligonucleotide. This may be converted into double stranded DNA by hybridization with a complementary sequence, or by polymerization with a DNA polymerase using the single strand as a template. One of skill would recognize that while chemical synthesis of DNA is limited to sequences of about 100 bases, longer sequences may be obtained by the ligation of shorter sequences.

Alternatively, subsequences may be cloned and the appropriate subsequences cleaved using appropriate restriction enzymes. The fragments may then be ligated to produce the desired DNA sequence.

In a preferred embodiment, DNA encoding fusion proteins of the present invention may be cloned using DNA amplification methods such as polymerase chain reaction (PCR). Thus, in a preferred embodiment, the V_{H} and V_{L} segments of 3B3 are PCR amplified, using primer pairs that introduce restriction sites (*e.g. Nde*I, *Hind*III, *etc.*) at the ends of the amplification product. The primers can be selected to introduce a linker between the V_{H} and V_{L} region (*e.g*. a 15 amino acid linker such as (Gly₄Ser)₃ SEQ ID NO:3). The amplified 3B3(Fv)-encoding nucleic acid can then be ligated into a plasmid encoding the cytotoxin (*e.g*. PE38) as described in Example 1.

Once a DNA sequence has been identified that encodes the desired 3B3 antibody (*see, e.g.,* SEQ ID NO:2), fusion proteins comprising that 3B3 antibody may be prepared by methods well known to those of skill in the art. The 3B3 antibody may be fused directly to the cytotoxin or may be joined indirectly to the cytotoxin through a peptide connector. The peptide connector may be present simply to provide space between the antibody and the cytotoxin or to facilitate mobility between these regions to enable them to each attain their optimum conformation. The DNA sequence comprising the connector may also provide sequences (such as primer sites or restriction sites) to facilitate cloning or may preserve the reading frame between the sequence encoding the targeting moiety and the sequence encoding the effector molecule. The design of such connector peptides will be well known to those of skill in the art. However, one particularly preferred connector is the peptide SGGPEGGS (SEQ ID NO:4), designated herein as the C3 connector.

Methods of producing fusion proteins are well known to those of skill in the art. Thus, for example, Chaudhary et al. (1989) Nature, 339: 394-397; Batra et al. (1990) J. Biol. Chem. 265: 15198-15202; Batra et al.(1989) Proc. Natl. Acad. Sci. USA, 86: 8545-8549; Chaudhary et al. (1990) Proc. Natl. Acad. Sci. USA 87: 1066-1070, describe the preparation of various single chain antibody-toxin fusion proteins.

Generally producing immunotoxin fusion proteins involves separately preparing the Fv light and heavy chains and DNA encoding any other protein to which they will be fused and recombining the DNA sequences in a plasmid or other vector to form a construct encoding the particular desired fusion protein. However, a simpler approach involves inserting the DNA encoding the particular Fv region into a construct already encoding the desired second protein.

One particularly preferred approach involves the use of plasmid pULI7 which encodes the B3(Fv)-PE38 immunotoxin (Benhar et al. (1994) Bioconjug. Chem., 5: 321-326). For each Fv, the V_{H} and V_{L} sequences are PCR amplified using the heavy chain and light chain in their respective plasmids as templates. The amplification primers are designed to have at their ends sequences that are complementary to the translation initiation, peptide linker and Fv-toxin junction (connector) which are common to the single-chain Fv-immunotoxin expression vectors. The PCR products are purified and annealed to a uracil-containing single stranded DNA corresponding to the pUL17 DNA prepared by rescue of pUL17 with a helper phage.

The annealed PCR products are extended using the single stranded DNA as a template (see, for example, MUTAGENE mutagenesis protocol, Biorad, Hercules, California, USA). The intact DNA may be used to transform cells and express the new fusion protein. Example 1 provides a detailed description of the preparation of 3B3(Fv)-PE38. The nucleic acid is constructed by spliced PCR using purified individual V_{H}- and V_{L}-PCR fragments of 3B3 and cloning them into the *Nde*I*-Hind*III site of pUL17. The vector contains the T7 promoter for expression in Studier's *E coli* BL21(λDE3) expression system (Studier et al. (1986) Mol. Biol. 189: 113-130).

The nucleic acid sequences encoding the fusion proteins may be expressed in a variety of host cells, including *E. coli,* other bacterial hosts, yeast, and various higher eukaryotic cells such as the COS, CHO and HeLa cells lines and myeloma cell lines. The recombinant protein gene will be operably linked to appropriate expression control sequences for each host. For *E. coli* this includes a promoter such as the T7, trp, or lambda promoters, a ribosome binding site and preferably a transcription termination signal. For eukaryotic cells, the control sequences will include a promoter and preferably an enhancer derived from immunoglobulin genes, SV40, cytomegalovirus, *etc.,* and a polyadenylation sequence, and may include splice donor and acceptor sequences.

The plasmids of the invention can be transferred into the chosen host cell by well-known methods such as calcium chloride transformation for *E*. *coli* and calcium phosphate treatment or electroporation for mammalian cells. Cells transformed by the plasmids can be selected by resistance to antibiotics conferred by genes contained on the plasmids, such as the amp, gpt, neo and hyg genes.

Once expressed, the recombinant fusion proteins can be purified according to standard procedures of the art, including ammonium sulfate precipitation, affinity columns, column chromatography, gel electrophoresis and the like (*see, generally,* Scopes, (1982) Protein Purification, Springer-Verlag, N.Y., Deutscher (1990) Methods in Enzymology Vol. 182: Guide to Protein Purification., Academic Press, Inc. N.Y.). Substantially pure compositions of at least about 90 to 95% homogeneity are preferred, and 98 to 99% or more homogeneity are most preferred for pharmaceutical uses. Once purified, partially or to homogeneity as desired, the polypeptides may then be used therapeutically.

One of skill in the art would recognize that after chemical synthesis, biological expression, or purification, the IL-13 receptor targeted fusion protein may possess a conformation substantially different than the native conformations of the constituent polypeptides. In this case, it may be necessary to denature and reduce the polypeptide and then to cause the polypeptide to re-fold into the preferred conformation. Methods of reducing and denaturing proteins and inducing re-folding are well known to those of skill in the art (See, Debinski et al. (1993) J. Biol. Chem., 268: 14065-14070; Kreitman and Pastan, Bioconjug. Chem., 4: 581-585; and Buchner et al. (1992) Anal. Biochem., 205: 263-270). Debinski *et al.,* for example, describe the denaturation and reduction of inclusion body proteins in guanidine-DTE. The protein is then refolded in a redox buffer containing oxidized glutathione and L-arginine. Detailed protocols for purification and refolding the expressed 3B3(Fv)-PE38 immunotoxin are provided in Example 1.

One of skill would recognize that modifications can be made to the gp120-targeted cytotoxins without diminishing their biological activity. Some modifications may be made to facilitate the cloning, expression, or incorporation of the targeting molecule into a fusion protein. Such modifications are well known to those of skill in the art and include, for example, a methionine added at the amino terminus to provide an initiation site, or additional amino acids placed on either terminus to create conveniently located restriction sites or termination codons.

### VII. Pharmaceutical Compositions.

The chimeric molecules of this invention are useful for parenteral, topical, oral, or local administration, such as by aerosol or transdermally, for prophylactic and/or therapeutic treatment. The pharmaceutical compositions can be administered in a variety of unit dosage forms depending upon the method of administration. For example, unit dosage forms suitable for oral administration include powder, tablets, pills, capsules and lozenges. It is recognized that the fusion proteins and pharmaceutical compositions of this invention, when administered orally, must be protected from digestion. This is typically accomplished either by complexing the protein with a composition to render it resistant to acidic and enzymatic hydrolysis or by packaging the protein in an appropriately resistant carrier such as a liposome. Means of protecting compounds from digestion are well known in the art *(see, e.g*., U.S. Patent 5,391,377 describing lipid compositions for oral delivery of therapeutic agents).

The pharmaceutical compositions of this invention are particularly useful for parenteral administration, such as intravenous administration or administration into a body cavity or lumen of an organ. The compositions for administration will commonly comprise a solution of the chimeric molecule (*e.g*., 3B3-PE38) dissolved in a pharmaceutically acceptable carrier, preferably an aqueous carrier. Pharmaceutically acceptable carriers can contain a physiologically acceptable compound that acts, for example, to stabilize the composition or to increase or decrease the absorption of the agent. Physiologically acceptable compounds can include, for example, carbohydrates, such as glucose, sucrose, or dextrans, antioxidants, such as ascorbic acid or glutathione, chelating agents, low molecular weight proteins, compositions that reduce the clearance or hydrolysis of the anti-mitotic agents, or excipients or other stabilizers and/or buffers.

Other physiologically acceptable compounds include wetting agents, emulsifying agents, dispersing agents or preservatives which are particularly useful for preventing the growth or action of microorganisms. Various preservatives are well known and include, for example, phenol and ascorbic acid. One skilled in the art would appreciate that the choice of a pharmaceutically acceptable carrier, including a physiologically acceptable compound depends, for example, on the rout of administration of the 3B3 immunotoxin and on the particular physio-chemical characteristics of the immunotoxin.

These pharmacological composition solutions are preferably sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of 3B3 immunotoxin in these formulations can vary widely, and will be selected primarily based on fluid volumes, viscosities, body weight and the like in accordance with the particular mode of administration selected and the patient's needs as described below.

### VIII. Treatment Regimen.

### A) 3B3 chimeric cytotoxin.

A typical pharmaceutical composition comprising a 3B3 immunotoxin (*e.g*. 3B3-PE38) for intravenous administration would be about 0.1 to 10 mg per patient per day. Dosages from 0.1 up to about 100 mg per patient per day may be used where the drug is well tolerated by the patient. Dosages may be calculated as ranging from 10 µg/kg up to 1 mg/kg, more preferably from about 100 µg/kg up to about 500 µg/kg depending on patient tolerance.

Actual methods for preparing parenterally administrable compositions will be known or apparent to those skilled in the art and are described in more detail in such publications as Remington's Pharmaceutical Science, 15th ed., Mack Publishing Company, Easton, Pennsylvania (1980).

The compositions containing the present fusion proteins or a cocktail thereof (*i.e*., with other therapeutics) can be administered for therapeutic treatments. In therapeutic applications, compositions are administered to a patient suffering from an HIV infection, in an amount sufficient to cure or at least partially arrest the disease and/or its complications. An amount adequate to accomplish this is defined as a "therapeutically effective dose." Amounts effective for this use will depend upon the severity of the disease and the general state of the patient's health.

Single or multiple administrations of the compositions may be administered depending on the dosage and frequency as required and tolerated by the patient. In any event, the composition should provide a sufficient quantity of the immunotoxins of this invention to effectively treat the patient.

It will be appreciated by one of skill in the art that there are some refuges for quiescent HIV infected cells (*e.g*. dendritic cells in lymph nodes). In some circumstances it may be preferred to provide direct administration of the 3B3 immunotoxin pharmaceutical to such sites (*e.g*. via injection). Alternatively, the therapeutic composition can be placed at the target site in a slow release formulation. Such formulations can include, for example, a biocompatible sponge or other inert or resorbable matrix material impregnated with the therapeutic composition, slow dissolving time release capsules or microcapsules, and the like. Typically a delivery catheter or time release formulation will be placed at the desired site as part of a surgical procedure.

### B) Combined therapies.

It is also contemplated that the 3B3 immunotoxins of this invention will be used in combination with other therapeutics including, but not limited to, reverse transcriptase inhibitors (*e.g*., AZT or ddI), and HIV protease inhibitors (*e.g*. Invirase, ritonavir (Norvir™), indiiavir (Crixivan™), *etc.).* Combined therapeutics provide a preferred modality in the treatment of HIV infection. A highly active antiretroviral therapy (HAART) showed encouraging results on reduction of viral loads in lymphoid tissues of HIV infected patients (Perelson et al. (1997) Nature 387: 188-191; Cavert et al. (1997) Science 276: 96-964). In this approach a cocktail consisting of a HIV protease inhibitor and two RIs (reverse transcriptase inhibitors) is administered for the treatment of HIV infected patients.

3B3 immunotoxins such as 3B3(Fv)-PE38 can have significant utility when used with existing multiple drug strategies. The therapeutic regimen for existing multiple drug strategies are well documented and known to those of skill in the art.

"Double-drug" therapy combining the 3B3 immunotoxins of this invention with a reverse transcriptase inhibitor or a protease inhibitor is expected to yield additional benefit. However, in a preferred embodiment, "triple-drug" therapy could be utilized to remove the soluble antigen, Env, and competing antibodies from the infected patient since viral titers and competing antibody titers crash following drug application. The immunotoxin of selected antibodies could then efficiently target HIV infected cells for destruction since they should still express Env on their surface since Env processing is performed by endogenous proteases. Temporary withdrawal of reverse transcriptase inhibitors while maintaining protease inhibitor dosing during 3B3(Fv)-PE38 dosing may facilitate targeting and elimination of infected cells. This can be a curative strategy if viral reservoirs are efficiently targeted and eliminated.

### IX. Therapeutic Kits.

In another embodiment, this invention provides for therapeutic kits. The kits include, but are not limited to an immunotoxin of this invention (*e.g*. 3B3-PE38) and/or a pharmaceutical composition thereof. The kits may also include other therapeutics (*e.g*., reverse transcriptase inhibitors, protease inhibitors, agents for the treatment of opportunistic infections) to be administered in a "multi-drug" therapeutic regimen.

The various compositions may be provided in separate containers for individual administration or for combination before administration. Alternatively the various compositions may be provided in a single container. The kits may also include various devices, buffers, assay reagents and the like for practice of the methods of this invention. In addition, the kits may contain instructional materials teaching the use of the kit in the various methods of this invention. While the instructional materials typically comprise written or printed materials they are not limited to such. Any medium capable of storing such instructions and communicating them to an end user is contemplated by this invention. Such media include, but are not limited to electronic storage media (*e.g*., magnetic discs, tapes, cartridges, chips), optical media (*e.g*., CD ROM), and the like. Such media may include addresses to internet sites that provide such instructional materials.

### EXAMPLES

The following examples are offered to illustrate, but not to limit the claimed invention.

### Example 1:.

In this study we have made, recombinant toxin containing the Fv portion of an antibody, 3B3, that has a high affinity and a broad cross-reactivity with many laboratory and clinical isolates of HIV. The parental antibody was isolated from a combinatorial phage display library constructed from bone marrow RNA of an infected individual (Burton et al. (1991) Proc. Natl. Acad. Sci. USA, 88: 10134-10137) and neutralizes many different laboratory strains of HIV-1 as well as many primary isolates (Kessler et al. (1997) Hum. Retrovinises 13: 575-582; Burton et al. (1994) Science 266: 1024-1027; Trkola et al. (1995) , J. Virol., 69: 6609-6617). The antibody reacts with the conserved CD4-binding site of gp120, the external subunit of the envelope glycoprotein (Barbas et al. (1994) Proc. Natl. Acad. Sci. USA, 91: 3809-3813).

### Materials and Methods

### Plasmid construction and production of recombinant protein.

The plasmid pAra-3B3 encodes the Fab fragment of antibody 3B3 directed against the gp120 glycoprotein of HIV-1 (Barbas et al. (1991) Proc. Natl. Acad. Sci. USA, 91: 3809-3813). DNA fragments encoding the Fv portion of the heavy and light chains of 3B3 antibody were obtained by PCR amplification using pAra-3B3 plasmid DNA as template. High fidelity Taq polymerase (Boehringer Mannheim) was used to avoid PCR errors. The primer pair used to amplify the heavy chain Fv region was T128 (5'-AAA CAT ATG CAG GTT CAG CTC GAG CAG TCT GGG GCT GAG GTG AAG AAG CCT GGG GCC TCA GTG AAG GTT TCT TGT CAG GCT-3', SEQ ID NO:5) and T129 (5'-TCC AGA TCC GCC ACC ACC TGA TCC GCC TCC GCC TGA GGA GAC GAT GAC CGT GGT CCC TTT GCC CCA GAC GTC-3',SEQ ID NO:6). The primer pair T-144 (5'-TCA GGT GGT GGC GGA TCT GGA GGT GGC GGA AGC GAC ATC GAG CTC ACG CAG TCT CCA GGC ACC CTG TCT CTG TCT CCA-3', SEQ ID NO:7) and T131 (5'-GGA AGC TTT CCT CTC CAG TTT GGT CCC CTG GCC AAA AGT GTA CGA GGA GGC ACC ATA-3', SEQ ID NO:8) was used to amplify the light chain Fv region. The plasmid PTFKB21.18 that encodes V_{H} and V_{L} domains of 3B3 antibody connected by a 15 amino acid linker and fused to PE38 was generated by spliced PCR using the purified individual V_{H} and V_{L}-PCR fragments using the primers T128 and T131 and was cloned into the *Nde*I-*Hind*III site of pULI7. The *Nde*I-*Hind*III sites fuses the inserted fragment in frame to the PE38, the truncated form of *Pseudomonas* exotoxin (Hwang et al. (1987) Cell, 48: 129-136). The vector contains the T7 promoter for expression in Studier's *E. coli* BL21(λDE3) expression system (Studier et al. (1986) Mol. Biol. 189: 113-130). The expression plasmid was confirmed to be correct by DNA sequencing on an ABI 373A sequencer using the dideoxy chain terminator sequencing kit.

The immunotoxin 3B3(Fv)PE38 was expressed in *E. coli* BL21(λDE3), and accumulated in inclusion bodies (IBs) and purified as active immunotoxin molecule following the method as previously described for other recombinant immunotoxins (Brinkmann et al. (1995) Methods 8: 143-195; Buchner et al. (1992) Anal. Biochem. 205: 263-270).

### Binding assays.

The affinities of 3B3(Fab) and 3B3(Fv) were assayed and compared by surface plasmon resonance (BIAcore, Pharmacia Biosensor) assay. Recombinant gp120 from HIV-1 MN strain was coupled to BIAcore sensor-chips (CM5, research grade, Pharmacia Biosensor) according to the manufacturer's specifications. The 3B3(Fab) and 3B3(Fv)-PE38 was applied to the chips and binding and dissociation (kₐₛₛ and k_{diss}) was determined from association and dissociation curves of the sensor grams with the BlAevaluation software package (Pharmacia Biosensor). K_{D} at equilibrium was calculated as K_{D} = k_{diss}/kₐₛₛ.

### Cytotoxicity assays.

The specific cytotoxicity of CD4-PE40, a fusion protein containing the HIV-binding portion of the human CD4 molecule linked to active regions of *Pseudomonas* exotoxin A and 3B3(Fv)-PE38 was assessed by protein synthesis inhibition assays on ENV15 cells (inhibition of incorporation of tritium labeled leucine into cellular protein) in 96-well plates as previously described (Brinkmann et al. (1991). Proc. Natl. Acad. Sci. USA, 88: 8616-8620).

ENV 15 cells were generated by transfecting the CHO cell line with a plasmid encoding HIV-1 envelope glycoprotein. The CHO cell line transfected with control plasmid was used as a negative control. The activity of the molecule is defined by the IC₅₀, the toxin concentration that reduces incorporation of radioactivity by 50%.

To determine the cell viability, cell Proliferation Reagent WST-1 (Boehringer Mannhein, Cat. No. 1644 807) assay was performed on HIV-1 chronically infected 8E5 cells (8E5/LAV AIDS Repository #95) and its uninfected parent line A3.01 (A3.01 AIDS Repository #166) cells. Experiments were carried out in 24-well plates. Wells were seeded with either 50,000 cells/well for A3.01 or 100,000 cells/well for 8E3 in 0.5 ml medium. Different amounts of immunotoxin were taken in 0.5 ml RPMI--10%FBS and added in each well and incubated for five days. The assays were performed by following the instructions provided with the kit.

### Stability assays.

The stability of the 3B3(Fv)-PE38 immunotoxins was determined by incubating them at 10:g/ml at 37°C in PBS containing 0.2% human serum albumin. Active immunotoxin remaining after incubation was determined by protein synthesis inhibition assays on ENV 15 cells.

### RESULTS

### Production and purification of 3B3(Fv)-PE38 immunotoxin.

To generate plasmids for the expression of 3B3(Fv)-PE38, a gene that codes for the V_{H} and V_{L} chain variable region of the antibody 3B3 separated by a fifteen amino acid linker was constructed by PCR using pAra-3B3 plasmid DNA as template. Schematics showing the immunotoxin fusion protein and the linear composition of the plasmid encoding 3B3(Fv)-PE38 immunotoxin are shown in Figure 1.

*E. coli* BL21 (λDE3) cells containing the plasmid pTKB21.18 for expression of the 3B3(Fv)-PE38 were grown and induced with IPTG. The fusion protein accumulated in insoluble intracellular inclusion bodies (IBs). These IBs contained almost pure recombinant protein, but in an insoluble and aggregated conformation. To generate protein with a native conformation, we solubilized and reduced the IBs GuCl and DTE, and refolded the protein by dilution in a buffer containing arginine as described in above (*see also* Brinkmann et al. (1995) Methods 8: 143-195; Buchner et al. (1992) Anal. Biochem. 205: 263-270). Refolded, soluble monomeric protein was then purified to near homogeneity from other bacterial and improperly folded proteins by ion exchange (Q-sepharose, Mono Q) and size exclusion chromatography. After the three column purification steps and using a standard refolding protocol for Fabs and immunotoxins, as described above, about 8% of the input protein was obtained as the monomeric scFv-immunotoxin.

### Binding affinity of 3b3(Fv)-PE38 immunotoxin towards gp120.

The binding affinity of the parental antibody 3B3 Fab, from which the 3B3(Fv) fragment was made, was improved by CDR walking mutagenesis using phage display technology (Barbas et al. (1991) Proc. Natl. Acad. Sci. USA, 91: 3809-3813). Frequently, scFvs possess lower binding affinity than the parental whole antibody or Fab fragment (Reiter et al. (1996) Nature Biotech. 14: 1239-1245). To investigate whether 3B3(Fv) retains the same binding affinity as the parental 3B3 Fab, the binding affinities of 3B3 Fab and 3B3 scFv immunotoxin were determined by surface plasmon resonance (BiaCore) assay. For the BiaCore assay the recombinant 3B3 Fab protein or 3B3(Fv)-PE38 was passed over gp120 treated sensor chips. The gp120 glycoprotein used was from HIV-1 MN strain. The association and dissociation rates are shown in Table 2. The K_{D} at binding equilibrium, calculated as K_{D} = k_{diss}/kₐₛₛ, was 36 nM for the 3B3 and 37 nM for the 3B3(Fv)-PE38 (Table 1). These data indicate that the 3B3(Fv) molecule has a binding affinity that approximates the parental 3B3 Fab antibody.

**Table 1. Affinity of Ara 3B3 Fab and 3B3 scFv-PE-38 immunotoxins. The affinity of Ara 3B3 Fab and 3B3scFv-PE38 was determined by surface plasmon resonance (BiaCore). Kₐₛₛ, k_{diss}, and kD (kD = k_{diss}/kₐₛₛ); binding at equilibrium) were calculated from the sensorgrams using the BIA evaluation software package.**

| Sample | Kₐₛₛ (M⁻¹ s⁻¹) | k_{diss} (s⁻¹) | K_{D} (M) |
|---|---|---|---|
| Ara 3B3 Fab | 0.76 x 10⁵ | 2.72 x 10⁻³ | 3.6 x 10⁻⁸ |
| 3B3 scFv-PE38 | 1.92 x 10⁵ | 7.03 x 10⁻³ | 3.7 x 10⁻⁸ |
| **Table 2.** Specific cytotoxicity of CD4-PE40 and 3B3scFv-PE38 immunotoxins. For ENV15 and CHO cell lines, cytotoxicity assays were performed by measuring incorporation of ³H-Leu into cellular proteins as described above. IC₅₀ is the concentration that causes 50% inhibition of protein synthesis after 20 hrs incubation with immunotoxin. For 8E5 and A3.01 cells, WST-1 cell viability assays were performed as described above. IC₅₀ is deduced from the concentration that causes 50% reduction of OD (A₄₅₀ nm) value after 5 days incubation with immunotoxin. | | | |
| | | IC₅₀, ng/ml | |

| cell line | gp120 | CD4-PE40 | 3B3scFv-PE38 |
|---|---|---|---|
| ENV15 | + | 40 | 2.5 |
| CHO | - | > 1000 | > 1000 |
| 8E5 | + | 90 | 2.1 |
| a3.01 | - | > 1000 | > 1000 |

### Specific Cytotoxicity of 3B3(Fv)-PE38 immunotoxin

Fusion proteins of antibody fragments with PE38 are cytotoxic to antigen positive cells that bind and internalize the fusion protein but are not cytotoxic to antigen negative cells. Two assay systems were employed to determine whether the 3B3(Fv)-immunotoxin was selectively internalized and translocated by cells expressing the HIV Env, leading to cytotoxicity. In the first assay, we used ENV15 cells, a transfected CHO cell line that expresses Env on its surface. CHO cell line transfected with control plasmid was used to determine the nonspecific killing of the immunotoxin. We also tested the CD4-PE40 immunotoxin, a chimeric protein containing CD4 attached to a truncated form of *Pseudomonas* exotoxin A (Chaudhary et al. (1988) Nature 335: 369-372), and compared its activity with 3B3(Fv)-PE38 immunotoxin.

As shown in Figure, 2A and Table 2, 3B3(Fv)-PE38 is about 116-fold more active on ENV15 cells than CD4-PE40. The IC₅₀ values for 3B3(Fv)-PE38 and CD4-PE40 on ENV15 cells are 2.5 and 40 ng/ml respectively. Both immunotoxins showed no cytotoxic activity on a CHO control cell line which does not express Env.

In the second assay, we used a human lymphocyte cell line 8E5 which is chronically infected by HIV, and the parental uninfected lymphocyte cell line, A3.01, as a negative control. The 8F-5 cells constitutively express Env and release infections HIV-1 particles. Figure 2B and Table 2 show that 3B3(Fv)-PE38 can kill 8E5 cells very effectively with an IC₅₀ of 2.1 ng/ml. In this assay, the immunotoxin is about 40-fold more active than CD4-PE40. Furthermore, the killing is specific because neither molecule has a cytotoxic effect on the HIV uninfected cell line at a concentration of 1,000 ng/ml.

### Stability 3B3(Fv)-PE38 immunotoxin.

Although Fvs are the smallest fractional modules that confer specific antigen binding, often Fv fragments by themselves are unstable. The hydrophobic residues on V_{H} and V_{L} domains, which are located at the heterodimer interface are insufficient to prevent dissociation of V_{L} and V_{H}. This results in aggregation and a reduction in binding affinity (Webbe et al. (1995) Immunol. 32:2 49-58). In most of the applications for which Fvs are used and in therapy in, particular, it is important that the Fvs are stable at 37°C in human serum so they will retain activity for a long time after injection into patients. To analyze if the 3B3(Fv) immunotoxin is stable, we assayed the stability of the 3B3(Fv)-PE38 in human serum albumin at 3°C.

The immunotoxin was incubated at 37°C for different periods of time in phosphate-buffered saline (PBS) containing 0.2% human serum albumin at a concentration of 10 :g/ml. The remaining activity of the immunotoxin was detected by a protein synthesis inhibition assay. The results of the stability analyses are shown in Table 3. The 3B3(Fv)-PE38 retains 80% of its activity even after 24 hrs. incubation at 37°C in. human serum albumin.

**Table 3. Stability of 3B3(Fv)-PE38 immunotoxin in human serum at 37°C. Immunotoxin 3B3(Fv)-PE38 was incubated with human serum albumin at a concentration of 10 :g/ml for the times shown at 37°C and then assayed for cytotoxic activity on ENV15 cells.**

| Time in hours | % activity remaining |
|---|---|
| 0 | 100 |
| 2 | 100 |
| 4 | 86 |
| 8 | 86 |
| 24 | 80 |

### DISCUSSION

The HIV antigen envelope glycoproteins (gp 120 and gp41) are the only viral proteins that are displayed on the HIV infected cell surface which can be recognized by specific antibodies. Since the gp120 is exposed on the cell surface of the infected cell, major efforts have been made to target the HIV infected cells by generating antibodies against the gp120 glycoprotein. Although there is a high degree of inter-isolate sequence variability of gp120, there are a few conserved regions, to which antibodies can be generated.

Antibody IgG1b12 was isolated from a combinatorial phage display library constructed from bone marrow RNA of an HIV infected individual (Burton et al. (1991) Proc. Natl. Acad. Sci. USA, 88: 10134-10137). This antibody is directed to the CD4 binding site of gp120 and unlike other monoclonal antibodies which are also directed to CD4-binding epitopes, it can neutralize many different laboratory strains of HIV-1 as well as many primary isolates (Kessler et al. (1997) Hum. Retrovinises 13: 575-582; Burton et al. (1994) Science 266: 1024-1027; Trkola et al. (1995) J. Virol., 69: 6609-6617).

Both the potency and breadth of neutralization activity of 3B3 were improved over the parental antibody IgClb12 (Barbas et al. (1994) Proc. Natl. Acad. Sci. USA, 91: 3809-3813). The resulting antibody, 3B3 thus has several characteristics which make it an attractive reagent for the targeted therapy of AIDS. We have used the Fv portion of antibody 3B3 to produce a recombinant immunotoxin, 3B3 (Fv)-PE38 and shown that this recombinant immunotoxin is very active and specific in killing gp120 expressing cells.

### Comparison of 3B3(Fv)-PE38 immunotoxin with other immunotoxins directed against HIV.

A number of different recombinant toxins and recombinant immunotoxins have been made which are directed against either HIV-1 antigens gp120 and gp41 (Chaudhary et al. (1988) Nature 335: 369-372; Till et al. Science 242: 1166-1168; Pincus et al. (1991) Immunol. 146: 4315-24; Pincus (1996) Antiviral Res., 33: 1-9) or against cell surface marker for T cells (Pincus (1996) Antiviral Res., 33: 1-9). The CD4-based toxins have been generated by fusing (Chaudhary et al. (1988) Nature 335: 369-372) or conjugating (Till et al. Science 242: 1166-1168) a region of the CD4 molecule containing the gp120 binding site to either the bacterial toxin PE40 or to the plant toxin ricin. The CD4-ricin conjugate can specifically and effectively kill HIV infected lymphocytes *in vitro* (Till et al. Science 242: 1166-1168). The immunotoxin anti-gp41 and ricin immunoconjugate, which is directed against gp41 of HIV has also been reported to be effective *in vitro* on HIV-infected T-cells and monocytes (Till et al. (1989) Proc. Natl. Acad. Sci. USA, 86: 1987-1991). But neither of these molecules have been examined in HIV-infected patients.

The most extensively studied chimeric toxin, CD4-PE40, was generated in our laboratory (Chaudhary et al. (1988) Nature 335: 369-372). It is a recombinant chimeric protein containing, CD4 linked to a 40,000 molecular weight form of *Pseudomonas* exotoxin A. CD4-PE40 was effective in killing an Env expressing cell line and chronically HIV-infected cells (Chaudhary et al. (1988) Nature 335: 369-372). Also when CD4-PE40 was used in combination with reverse transcriptase inhibitors that block the viral replication cycle, a synergistic effect was observed, leading to elimination of infectious HIV from human T-cell cultures (Ashorn et al. (1990) Proc. Natl. Acad. Sci. USA, 87: 8889-8893). Based on these data preclinical development of CD4-PE40 was carried out and it was found to be very well tolerated by monkeys so that 250 :g/kg could be administered daily for 10 days without serious toxicity. Subsequently phase I clinical trials were performed (Ramachandran et al. (1994) J. Infect. Dis. 170: 1009-1013; Davey et al. (1994) Infect. Dis. 170: 1180-1188). Surprisingly, CD4-PE40 demonstrated very high toxicity in infected patients with a maximum tolerated dose of only 10 :g/kg.

The major side effect was liver toxicity. No evidence of anti-HIV effect of this protein in this trial was obtained, probably because of the low amount of the drug that could be given to patients. The toxicity of CD4-PE40 is believed to be due to the CD4 portion directing the immunotoxin to the liver, since we have subsequently given several other recombinant immunotoxins to patients at doses of up to 50 :g/kg without observing dose limiting liver toxicity. In addition a chemical conjugate of PE38 with a whole monoclonal antibody has been given in doses up to 100 :g/kg without liver toxicity (Pai et al. (1996) Nature Med- 2:3 50-353). 3B3(Fv)-PE38 is about 20- to 30-fold more effective in killing gp120 expressing and HIV- infected cells *in vitro* than CD4-PE40 and should be devoid of the nonspecific toxicity observed with CD4-FE40.

We believe that immunotoxins such as 3B3(Fv)-PE38 can have significant utility when used with existing multiple drug strategies. "Triple-drug" therapy could be utilized to remove the soluble antigen, Env, and competing antibodies from the infected patient since viral titers and competing antibody titers crash following their application. The immunotoxin of selected antibodies could then efficiently target HIV infected cells for destruction since they should still express Env on their surface since Env processing is performed by endogenous proteases. Temporary withdrawal of reverse transcriptase inhibitors while maintaining protease inhibitor dosing during 3B3(Fv)-PE38 dosing may facilitate targeting and elimination of infected cells. This could be a curative strategy if viral reservoirs could be efficiently targeted and eliminated. Thus, we believe 3B3(Fv)-PE38 is a strong candidate for use in the treatment of HIV disease.

## Claims

1. A single-chain Fv antibody, a disulfide-stabilized Fv antibody, or a single-chain Fab antibody, which antibody has the binding specificity of 3B3(Fv) of SEQ ID NO:1.

2. The antibody of claim 1, wherein said antibody has the amino acid sequence of 3B3(Fv) of SEQ ID NO: 1 or conservative substitutions thereof.

3. The antibody of claim 2, wherein said antibody is 3B3(Fv) of SEQ ID NO: 1.

4. A nucleic acid that encodes a single-chain Fv antibody of claim 1, 2 or 3.

5. An immunotoxin comprising a cytotoxin attached to an antibody of claim 1, 2 or 3, wherein said immunotoxin specifically binds to and kills mammalian cells infected with HIV-1.

6. The immunotoxin of claim 5, wherein said cytotoxin is selected from ricin, abrin, a modified diphtheria toxin, and a modified *Pseudomonas* exotoxin.

7. The immunotoxin of claim 6, wherein said cytotoxin is a modified *Pseudomonas* exotoxin.

8. A nucleic acid that encodes a single-chain fusion protein immunotoxin, said nucleic acid comprising:
a) a nucleic acid sequence that encodes a single-chain antibody of claim 1, 2 or 3; and
b) a nucleic acid sequence that encodes a modified *Pseudomonas* exotoxin.

9. A pharmaceutical composition comprising a pharmaceutically acceptable carrier or excipient and an immunotoxin comprising a modified *Pseudomonas* exotoxin attached to an antibody of claim 1, 2 or 3, wherein said immunotoxin specifically binds to and kills mammalian cells infected with HIV-1.

10. A method of killing a cell displaying a gp120 protein or fragment thereof, said method comprising contacting said cell *ex vivo* with an immunotoxin comprising a modified *Pseudomonas* exotoxin attached to an antibody of claim 1, 2 or 3, wherein said immunotoxin specifically binds to and kills mammalian cells infected with HIV-1.

11. An immunotoxin comprising a modified *Pseudomonas* exotoxin attached to an antibody of claim 1, 2 or 3, for use in a method of killing or inhibiting the growth of cells bearing gp120 protein or fragment thereof by administering said immunotoxin to an organism containing said cells in an amount sufficient to kill or inhibit the growth of said cells, wherein said immunotoxin specifically binds to and kills mammalian cells infected with HIV-1.

12. The immunotoxin of claim 7, the nucleic acid of claim 8, the pharmaceutical composition of claim 9, the method of claim 10 or the immunotoxin of claim 11, wherein said modified *Pseudomonas* exotoxin is selected from PE38, PE40, PE38KDEL and PE38REDL.

13. The immunotoxin, nucleic acid, pharmaceutical composition or method of claim 12, wherein said modified *Pseudomonas* exotoxin is PE38.

14. The immunotoxin of any one of claims 5 to 7, the nucleic acid of claim 8, the pharmaceutical composition of claim 9, the method of claim 10 or the immunotoxin of claim 11, wherein said antibody is a recombinantly expressed single-chain Fv.

15. The immunotoxin of any one of claims 5 to 7, the pharmaceutical composition of claim 9, the method of claim 10 or the immunotoxin of claim 11, wherein said immunotoxin is a fusion protein.

16. The immunotoxin of claim 7, the nucleic acid of claim 8, the pharmaceutical composition of claim 9, the method of claim 10 or the immunotoxin of claim 11, wherein said immunotoxin is 3B3(Fv)-PE38.

17. The immunotoxin of claim 5, 6 or 7, wherein said immunotoxin is suspended or dissolved in a pharmaceutically acceptable carrier or excipient.

18. The immunotoxin of claim 11, for use in a said method further comprising administering to said organism a protease inhibitor.

19. The immunotoxin of claim 11, for use in a said method further comprising administering to said organism a reverse transcriptase inhibitor.

20. The immunotoxin of claim 11, for use in a said method further comprising administering to said organism both a protease inhibitor and a reverse transcriptase inhibitor and then withdrawing the reverse transcriptase inhibitor while maintaining protease inhibitor dosing during administration of said immunotoxin.

21. A kit for killing cells that display a gp120 protein, said kit comprising a container containing an immunotoxin as claimed in any one of claims 5 to 7 and 11 to 17.

22. Use of an immunotoxin as claimed in any one of claims 5 to 7 and 11 to 17 for the manufacture of a medicament for killing or inhibiting the growth of cells bearing gp120 protein or a fragment thereof.

23. The use of claim 22 wherein the medicament is for use in combination with a reverse transcriptase inhibitor or a protease inhibitor.

## Patentansprüche

1. Einzelkettiger Fv-Antikörper, Disulfid-stabilisierter Fv-Antikörper oder einzelkettiger Fab-Antikörper, welcher Antikörper die Bindungsspezifität des 3B3(Fv) der SEQ ID NR. 1 aufweist.

2. Antikörper nach Anspruch 1, wobei der Antikörper die Aminosäuresequenz des 3B3(Fv) der SEQ ID NR. 1 oder konservative Substitutionen davon aufweist.

3. Antikörper nach Anspruch 2, wobei der Antikörper 3B3(Fv) der SEQ ID NR. 1 ist.

4. Nukleinsäure, die einen einzelkettigen Fv-Antikörper nach Anspruch 1, 2 oder 3 codiert.

5. Immuntoxin, umfassend ein Cytotoxin in Bindung an einen Antikörper nach Anspruch 1, 2 oder 3, wobei das Immuntoxin an Säugerzellen, die mit HIV-1 infiziert sind, spezifisch bindet und sie abtötet.

6. Immuntoxin nach Anspruch 5, wobei das Cytotoxin ausgewählt ist aus Ricin, Abrin, einem modifizierten Diphtherie-Toxin und einem modifizierten Pseudomonas-Exotoxin.

7. Immuntoxin nach Anspruch 6, wobei das Cytotoxin ein modifiziertes Pseudomonas-Exotoxin ist.

8. Nukleinsäure, die ein einzelkettiges Fusionsprotein-Immuntoxin codiert, welche Nukleinsäure umfasst:
a) eine Nukleinsäuresequenz, die einen einzelkettigen Antikörper nach Anspruch 1, 2 oder 3 codiert; und
b) eine Nukleinsäuresequenz, die ein modifiziertes *Pseudomonas*-Exotoxin codiert.

9. Pharmazeutische Zusammensetzung, umfassend einen pharmazeutisch akzeptablen Träger oder Exzipienten und ein Immuntoxin, umfassend ein modifiziertes Pseudomonas-Exotoxin in Bindung an einen Antikörper nach Anspruch 1, 2 oder 3, wobei das Immuntoxin an Säugerzellen, die mit HIV-1 infiziert sind, spezifisch bindet und sie abtötet.

10. Methode zum Abtöten einer Zelle, die ein gp120-Protein oder Fragment davon aufweist, welche Methode das Kontaktieren der Zelle *ex vivo* mit einem Immuntoxin, umfassend ein modifiziertes Pseudomonas-Exotoxin in Bindung an einen Antikörper nach Anspruch 1, 2 oder 3, wobei das Immuntoxin an Säugerzellen, die mit HIV-1 infiziert sind, spezifisch bindet und sie abtötet, umfasst.

11. Immuntoxin, umfassend ein modifiziertes Pseudomonas-Exotoxin in Bindung an einen Antikörper nach Anspruch 1, 2 oder 3, zur Verwendung bei einer Methode zum Abtöten oder Hemmen des Wachstums von Zellen, die ein gp120-Protein oder Fragment davon aufweisen, durch Verabreichen des Immuntoxins an einen Organismus, der diese Zellen enthält, in einer Menge, die ausreicht, um diese Zellen abzutöten oder ihr Wachstum zu hemmen, wobei das Immuntoxin an Säugerzellen, die mit HIV-1 infiziert sind, spezifisch bindet und sie abtötet.

12. Immuntoxin nach Anspruch 7, Nukleinsäure nach Anspruch 8, pharmazeutische Zusammensetzung nach Anspruch 9, Methode nach Anspruch 10 oder Immuntoxin nach Anspruch 11, wobei das modifizierte Pseudomonas-Exotoxin ausgewählt ist aus PE38, PE40, PE38KDEL und PE38REDL.

13. Immuntoxin, Nukleinsäure, pharmazeutische Zusammensetzung oder Methode nach Anspruch 12, wobei das modifizierte *Pseudomonas*-Exotoxin PE38 ist.

14. Immuntoxin nach einem der Ansprüche 5 bis 7, Nukleinsäure nach Anspruch 8, pharmazeutische Zusammensetzung nach Anspruch 9, Methode nach Anspruch 10 oder Immuntoxin nach Anspruch 11, wobei der Antikörper ein rekombinant exprimiertes einzelkettiges Fv ist.

15. Immuntoxin nach einem der Ansprüche 5 bis 7, pharmazeutische Zusammensetzung nach Anspruch 9, Methode nach Anspruch 10 oder Immuntoxin nach Anspruch 11, wobei das Immuntoxin ein Fusionsprotein ist.

16. Immuntoxin nach Anspruch 7, Nukleinsäure nach Anspruch 8, pharmazeutische Zusammensetzung nach Anspruch 9, Methode nach Anspruch 10 oder Immuntoxin nach Anspruch 11, wobei das Immuntoxin 3B3(Fv)-PE38 ist.

17. Immuntoxin nach Anspruch 5, 6 oder 7, wobei das Immuntoxin in einem pharmazeutisch geeigneten Träger oder Exzipienten suspendiert oder gelöst ist.

18. Immuntoxin nach Anspruch 11, zur Verwendung bei einer solchen Methode, die außerdem das Verabreichen an den Organismus eines Protease-Inhibitors umfasst.

19. Immuntoxin nach Anspruch 11, zur Verwendung bei einer solchen Methode, die außerdem das Verabreichen an den Organismus eines Reverse Transkriptase-Inhibitors umfasst.

20. Immuntoxin nach Anspruch 11, zur Verwendung bei einer solchen Methode, die außerdem das Verabreichen an den Organismus sowohl eines Protease-Inhibitors als auch eines Reverse Transkriptase-Inhibitors und dann Entziehen des Reverse Transkriptase-Inhibitors unter Aufrechterhaltung der Dosierung des Protease-Inhibitors während der Verabreichung des Immuntoxins umfasst.

21. Kit zum Abtöten von Zellen, die ein gp120-Protein aufweisen, welches Kit einen Behälter umfasst, der ein Immuntoxin nach einem der Ansprüche 5 bis 7 und 11 bis 17 enthält.

22. Verwendung eines Immuntoxins nach einem der Ansprüche 5 bis 7 und 11 bis 17 für die Herstellung eines Medikaments zum Abtöten oder Hemmen des Wachstums von Zellen, die das gp120-Protein oder ein Fragment davon tragen.

23. Verwendung nach Anspruch 22, wobei das Medikament zur Verwendung in Kombination mit einem Reverse Transkriptase-Inhibitor oder einem Protease-Inhibitor ist.

## Revendications

1. Anticorps Fv simple chaîne, anticorps Fv stabilisé par une liaison disulfure, ou anticorps Fab simple chaîne, lequel anticorps possède la spécificité de liaison de 3B3(Fv) de SEQ ID NO: 1.

2. Anticorps selon la revendication 1, où ledit anticorps possède la séquence d'acides aminés de 3B3 (Fv) de SEQ ID NO: 1 ou des substitutions conservatrices de celle-ci.

3. Anticorps selon la revendication 2, où ledit anticorps est 3B3(Fv) de SEQ ID NO: 1.

4. Acide nucléique qui code pour un anticorps Fv simple chaîne selon la revendication 1, 2 ou 3.

5. Immunotoxine comprenant une cytotoxine attachée à un anticorps selon la revendication 1, 2 ou 3, où ladite immunotoxine se lie spécifiquement à et tue des cellules mammaliennes infectées par le VIH-1.

6. Immunotoxine selon la revendication 5, où ladite cytotoxine est sélectionnée parmi la ricine, l'abrine, une toxine diphtérique modifiée et une exotoxine de *Pseudomonas* modifiée.

7. Immunotoxine selon la revendication 6, où ladite cytotoxine est une exotoxine de *Pseudomonas* modifiée.

8. Acide nucléique qui code pour une immunotoxine protéine de fusion simple chaîne, ledit acide nucléique comprenant :
a) une séquence d'acide nucléique qui code pour un anticorps simple chaîne selon la revendication 1, 2 ou 3 ; et
b) une séquence d'acide nucléique qui code pour une exotoxine de *Pseudomonas* modifiée.

9. Composition pharmaceutique comprenant un véhicule ou un excipient pharmaceutiquement acceptable et une immunotoxine comprenant une exotoxine de *Pseudomonas* modifiée attachée à un anticorps selon la revendication 1, 2 ou 3, où ladite immunotoxine se lie spécifiquement à et tue des cellules mammaliennes infectées par le VIH-1.

10. Procédé pour tuer une cellule présentant une protéine gp120 ou un fragment de celle-ci, ledit procédé consistant à mettre en contact ladite cellule *ex vivo* avec une immunotoxine comprenant une exotoxine de *Pseudomonas* modifiée attachée à un anticorps selon la revendication 1, 2 ou 3, où ladite immunotoxine se lie spécifiquement à et tue des cellules mammaliennes infectées par le VIH-1.

11. Immunotoxine comprenant une exotoxine de *Pseudomonas* modifiée attachée à un anticorps selon la revendication 1, 2 ou 3, destinée à être utilisée dans un procédé pour tuer ou inhiber la croissance de cellules portant une protéine gp120 ou un fragment de celle-ci, en administrant ladite immunotoxine à un organisme contenant lesdites cellules en une quantité suffisante pour tuer ou inhiber la croissance desdites cellules, où ladite immunotoxine se lie spécifiquement à et tue des cellules mammaliennes infectées par le VIH-1.

12. Immunotoxine selon la revendication 7, acide nucléique selon la revendication 8, composition pharmaceutique selon la revendication 9, procédé selon la revendication 10 ou immunotoxine selon la revendication 11, où ladite exotoxine de *Pseudomonas* modifiée est sélectionnée parmi PE38, PE40, PE38KDEL et PE38REDL.

13. Immunotoxine, acide nucléique, composition pharmaceutique ou procédé selon la revendication 12, où ladite exotoxine de *Pseudomonas* modifiée est PE38.

14. Immunotoxine selon l'une quelconque des revendications 5 à 7, acide nucléique selon la revendication 8, composition pharmaceutique selon la revendication 9, procédé selon la revendication 10 ou immunotoxine selon la revendication 11, où ledit anticorps est un Fv simple chaîne exprimé de manière recombinante.

15. Immunotoxine selon l'une quelconque des revendications 5 à 7, composition pharmaceutique selon la revendication 9, procédé selon la revendication 10 ou immunotoxine selon la revendication 11, où ladite immunotoxine est une protéine de fusion.

16. Immunotoxine selon la revendication 7, acide nucléique selon la revendication 8, composition pharmaceutique selon la revendication 9, procédé selon la revendication 10 ou immunotoxine selon la revendication 11, où ladite immunotoxine est 3B3(Fv)-PE38.

17. Immunotoxine selon la revendication 5, 6 ou 7, où ladite immunotoxine est en suspension ou est dissoute dans un véhicule ou un excipient pharmaceutiquement acceptable.

18. Immunotoxine selon la revendication 11, destinée à être utilisée dans un dit procédé consistant en outre à administrer audit organisme un inhibiteur de protéase.

19. Immunotoxine selon la revendication 11, destinée à être utilisée dans un dit procédé consistant en outre à administrer audit organisme un inhibiteur de transcriptase inverse.

20. Immunotoxine selon la revendication 11, destinée à être utilisée dans un dit procédé consistant en outre à administrer audit organisme à la fois un inhibiteur de protéase et un inhibiteur de transcriptase inverse, puis à ensuite retirer l'inhibiteur de transcriptase inverse tout en maintenant le dosage de l'inhibiteur de protéase pendant l'administration de ladite immunotoxine.

21. Kit pour tuer des cellules qui présentent une protéine gp120, ledit kit comprenant un récipient contenant une immunotoxine selon l'une quelconque des revendications 5 à 7 et 11 à 17.

22. Utilisation d'une immunotoxine selon l'une quelconque des revendications 5 à 7 et 11 à 17, pour la fabrication d'un médicament destiné à tuer ou à inhiber la croissance de cellules portant une protéine gp120 ou un fragment de celle-ci.

23. Utilisation selon la revendication 22, où le médicament est destiné à être utilisé en combinaison avec un inhibiteur de transcriptase inverse ou un inhibiteur de protéase.
